# EUROPEAN PATENT SPECIFICATION

(11) **EP 3 658 081 B1**
(45) Date of publication and mention of the grant of the patent: **21.02.2024**
(21) Application number: 18839359.9
(22) Date of filing: 27.07.2018
(51) Int. Cl.: A61F 2/88, A61F 2/04

(54) **IMPLANT FOR EXPANDING THE DIAMETER OF THE PROSTATIC URETHRA**
IMPLANTAT ZUR ERWEITERUNG DES DURCHMESSERS DER PROSTATISCHEN HARNRÖHRE
IMPLANT POUR AUGMENTER LE DIAMÈTRE DE L'URÈTRE PROSTATIQUE

(30) Priority: 28.07.2017 US 201762537963 P
(43) Date of publication of application: 03.06.2020
(73) Proprietor: Zenflow, Inc., San Francisco, California 94080 (US)
(72) Inventor: BLY, Austin Michael, San Clemente California 92673 (US); CHAMBERS, Matthew, San Francisco California 94080 (US); DAMIANO, Nicholas Ralph, San Francisco California 94131 (US); JABBA, Ronald J., Redwood City California 94062 (US); MEHTA, Shreya, San Francisco California 94110 (US); SICOTTE, Marcel Song, San Francisco California 94109 (US); WEISS, Aaron M., Oakland California 94602 (US)
(74) Representative: Booth, Catherine Louise
(86) International application number: PCT/US2018/044180
(87) International publication number: WO 2019/023633

(56) References cited:
- WO-A1-2005/016185
- WO-A1-2018/107123
- US-A- 5 514 178
- US-A- 5 540 713
- US-A1- 2008 183 268
- US-A1- 2009 177 288
- US-A1- 2011 077 676
- US-A1- 2015 257 908
- US-B2- 7 112 226

## Description

### FIELD

The subject matter described herein relates to systems, devices, and methods for expansion of a body lumen, particularly, expansion of the prostatic urethra with an implantable medical device.

Prior art implants for expanding a body lumen and deployment devices for such implants are disclosed in WO2005/016185, US2008/0183268, US5540713 and US2015/0257908.

### BACKGROUND

There are several clinical reasons for placement of an implant into the prostatic urethra, including treatment of urinary retention associated with benign prostatic hyperplasia (BPH or enlarged prostate), blockages from prostate cancer, bladder cancer, urinary tract injury, prostatitis, bladder sphincter dyssynergia, and benign or malignant urethral stricture. Due to the naturally complex anatomical geometry, multi-lobular nature of the prostate, patient-to-patient geometric and tissue variability, and anatomical restrictions associated with those conditions, implants such as cylindrical mesh, polymer, or dense coil stents have resulted in short-term and long-term complications including migration, encrustation, hyperplastic ingrowth, incontinence, ejaculatory dysfunction, pain or discomfort, and difficult removal. Specifically, encrustation and stone formation can readily occur in some patients due to excessive exposure to static urine or flow stagnation caused by junctions, sharp angles, or irregular surface geometry. Hyperplastic ingrowth can result from injury to the urethral mucosa caused by sharp edges or irritative materials. Migration can occur when implants are not properly fixed in the urethra and are subjected to the repeated internal stresses of voiding, ejaculation, or external stresses. When adverse symptoms recur or new symptoms appear, removal of the implant is often desired. Implants that are composed of multiple wires or parts that reside within the prostatic tissue rather than in the prostatic urethral lumen are often difficult to remove, which has led to reduced usage of such devices by urologists. Owing to the unique geometry of the prostatic urethra, a low-profile implant that minimizes urine exposure and remains in its intended position is desired, yet such an implant must also retract the obstructing prostatic lobes sufficiently to relieve symptoms.

Accordingly, needs exist for systems, devices, and methods that overcome these or other deficiencies in treating undesirable conditions, such as BPH.

### SUMMARY

The aspects and/or embodiments and/or examples disclosed in the following description but that are not covered by the appended claims are considered as not being part of the present invention and are disclosed by way of example only. Provided herein are a number of example embodiments of implants for maintaining a prostatic urethra lumen in an at least partially open state. Embodiments of the implant can include an elastic main body that can be formed from a single wire. The elastic main body can be biased to laterally expand and longitudinally contract towards an at-rest configuration for maintaining patency of the lumen. The elastic main body in the at-rest configuration has multiple expander structures (e.g., ring-shaped structures) that are non-coplanar, the ring-shaped structures located about a longitudinal axis of the implant. Each of the multiple expander structures can be coupled with another of the expander structures by an interconnect portion of the elastic main body. The interconnect portion can extend longitudinally such that the expander structures are spaced apart.

Numerous variations in configurations of the implant are described, including variations in shapes of the expander structures, variations in configurations of engagement features, variations in radial coverage provided by the implant, variations in symmetry, variations in placement of the embodiments, the inclusion of longitudinal projections and anchors, and others. Methods of implantation and manufacture are also described but are not part of the invention.

Other systems, devices, methods, features and advantages of the subject matter described herein will be or will become apparent to one with skill in the art upon examination of the following figures and detailed description. The invention is directed to an implant for placement in a prostatic urethra lumen as defined in claim 1. Dependent claims 2 - 12 define particular embodiments.

### BRIEF DESCRIPTION OF THE FIGURES

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely.
FIG. 1A is a block diagram depicting an example embodiment of a delivery system.
FIGs. 1B, 1C, and 1D are side, end, and perspective views, respectively, depicting an example embodiment of an implant.
FIGs. 2A-2H are perspective views depicting example embodiments of a delivery system in different stages of deployment of an implant.
FIGs. 3A-3C are perspective views depicting example embodiments of a grasper component in use within a delivery system.
FIG. 4 is a flowchart depicting an example embodiment of a method for delivering an implant.
FIG. 5A as an example cross-section of the male anatomy.
FIG. 5B is an example cross-section of the male anatomy having an example embodiment of an implant deployed therein.
FIG. 5C is an example sagittal cross-section of the male anatomy and FIG. 5D is an example cross-section of the male anatomy taken along line 5D-5D of FIG. 5C.
FIG. 5E is an example cross-section of the male anatomy having an example embodiment of an implant deployed therein and FIG. 5F is an example cross-section of the male anatomy taken along line 5F-5F of FIG. 5E.
FIG. 5G is an example cross-section of the male anatomy having an example embodiment of an implant deployed therein and FIG. 5H is an example cross-section of the male anatomy taken along line 5H-5H of FIG. 5G.
FIGs. 6A and 6B are perspective views depicting additional example embodiments of an implant.
FIG. 6C is an end-on view depicting the embodiment of FIG. 6A within a prostatic urethra.
FIG. 6D is an end-on view depicting an example embodiment of an implant within a prostatic urethra.
FIG. 6E is a perspective view depicting an example embodiment of an implant within a prostatic urethra.
FIG. 6F is an end-on view depicting a distal-most ring-shaped structure of the example embodiment of FIG. 6E.
FIGs. 7A and 7B are perspective and end-on views, respectively, depicting an example embodiment of an implant.
FIGs. 7C and 7D are perspective and end-on views, respectively, depicting an example embodiment of an implant.
FIG. 8A is a perspective view depicting an example embodiment of an implant according to the invention.
FIGs. 8B and 8C are end-on views of the example embodiment of FIG. 8A within a prostatic urethra.
FIGs. 8D-8H are end-on views depicting example embodiments of implants.
FIGs. 9A and 9B are perspective and end-on views, respectively, depicting an example embodiment of an implant.
FIGs. 9C and 9D are perspective and end-on views, respectively, depicting an example embodiment of an implant.
FIGs. 9E and 9F are perspective and end-on views, respectively, depicting an example embodiment of an implant.
FIGs. 9G and 9H are perspective and end-on views, respectively, depicting an example embodiment of an implant.
FIGs. 9I and 9J are perspective and end-on views, respectively, depicting an example embodiment of an implant.
FIGs. 10A and 10B are perspective and sagittal views, respectively, depicting an example embodiment of an implant.
FIG. 10C is a perspective view depicting another example embodiment of an implant.
FIG. 10D is a sagittal view depicting the embodiment of FIG. 10C within a prostatic urethra.
FIG. 10E is a perspective view depicting an example embodiment of an implant.
FIGs. 11A and 11B are perspective views depicting additional example embodiments of an implant.
FIGs. 11C and 11D are sagittal cross-sectional views depicting example embodiments of an implant within a prostatic urethra.
FIG. 11E is a perspective view depicting an example embodiment of an implant.
FIG. 12A is a perspective view depicting an example embodiment of an implant within a prostatic urethra and bladder neck.

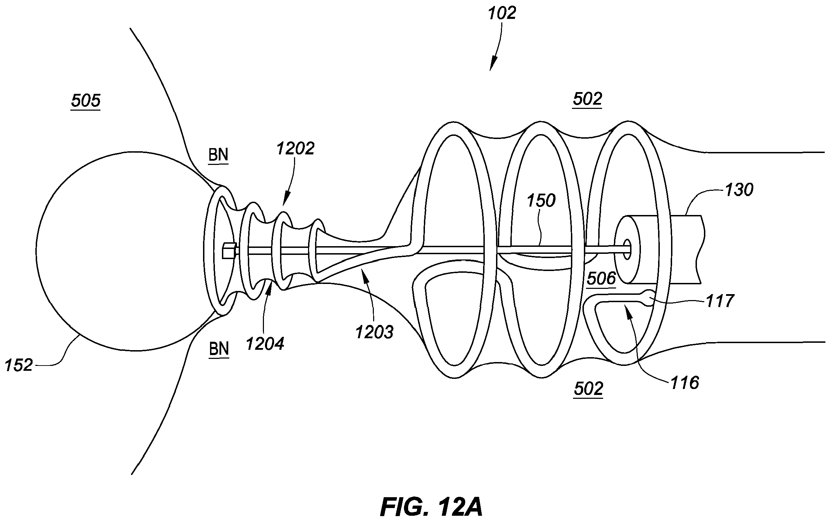

FIG. 12A is a perspective view depicting an example embodiment of an implant within a prostatic urethra and bladder neck.

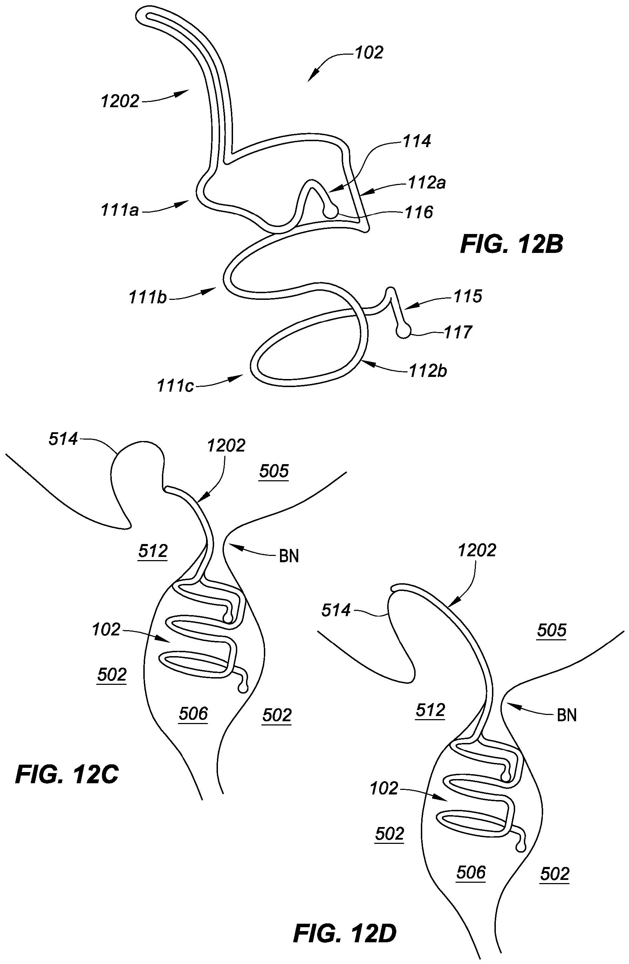

FIG. 12B is a perspective view depicting another example embodiment of an implant.
FIGs. 12C and 12D are side views depicting example embodiments of the implant in a sagittal cross-section of the urethra and bladder.

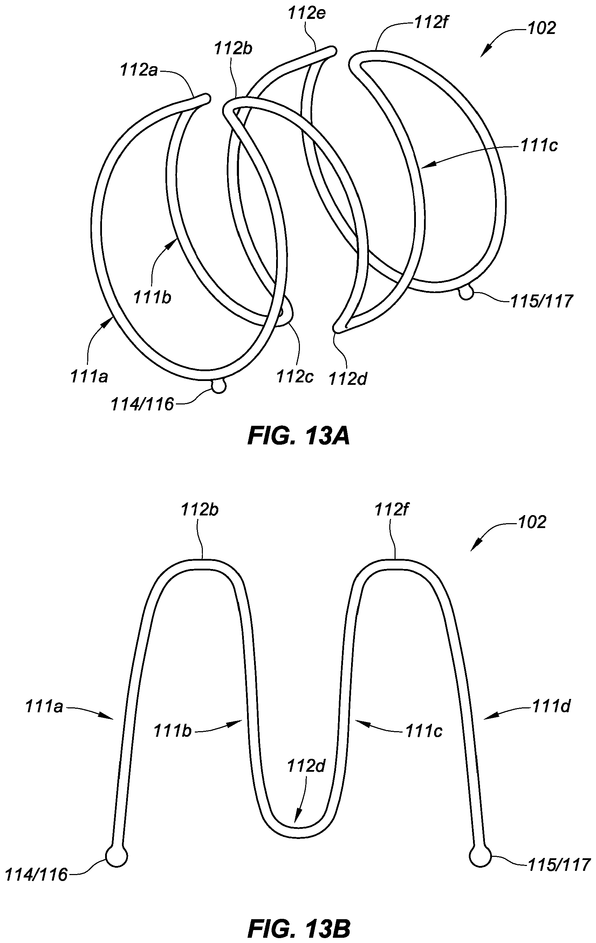

FIGs. 13A and 13B are perspective and side views, respectively, depicting another example embodiment of an implant.

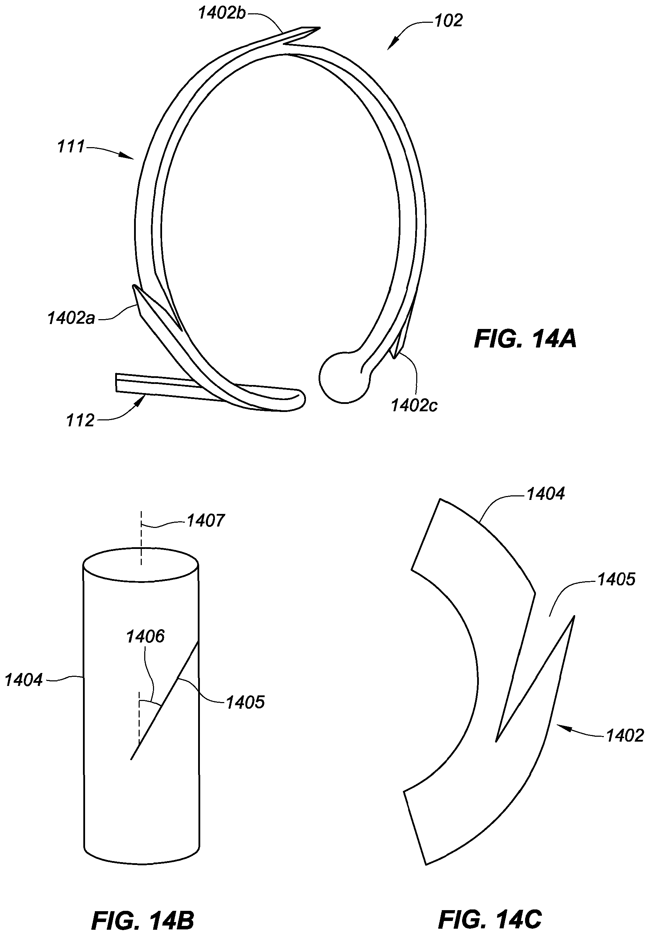

FIG. 14A is a perspective view depicting an example embodiment of a ring-shaped structure with anchors.
FIGs. 14B and 14C are diagrams depicting a portion of an implant body during manufacture and deployment, respectively.

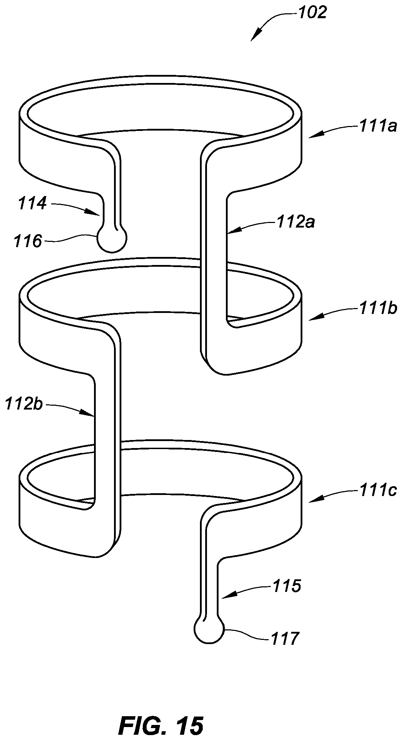

FIG. 15 is a perspective view depicting an example embodiment of an implant.

### DETAILED DESCRIPTION

Before the present subject matter is described in detail, it is to be understood that this disclosure is not limited to the particular embodiments described, as such may, of course, vary. It is also to be understood that the terminology used herein is for the purpose of describing particular embodiments only, and is not intended to be limiting, since the scope of the present disclosure will be limited only by the appended claims.

The subject matter presented herein is described in the context of delivery or deployment of one or more implants within the prostatic urethra. The purpose for deployment of the implant(s) in the prostatic urethra can vary. The embodiments described herein are particularly suited for treatment of BPH, but they are not limited to such. Other conditions for which these embodiments can be used include, but are not limited to, treatment of blockages from prostate cancer, bladder cancer, urinary tract injury, prostatitis, bladder sphincter dyssynergia, benign or malignant urethral stricture, voiding dysfunction related to spinal cord injury, bladder neck contractures or stenosis. Further, these embodiments can have applicability for deployment of one or more implants in other locations of the urinary tract (e.g., ureter) or in the bladder, as well as other biological lumens, cavities, or spaces, such as the human vasculature, respiratory tract, cardiac system, pulmonary system, or gastro-intestinal tract, including locations within the heart, stomach, intestines, liver, spleen, pancreas, and kidney.

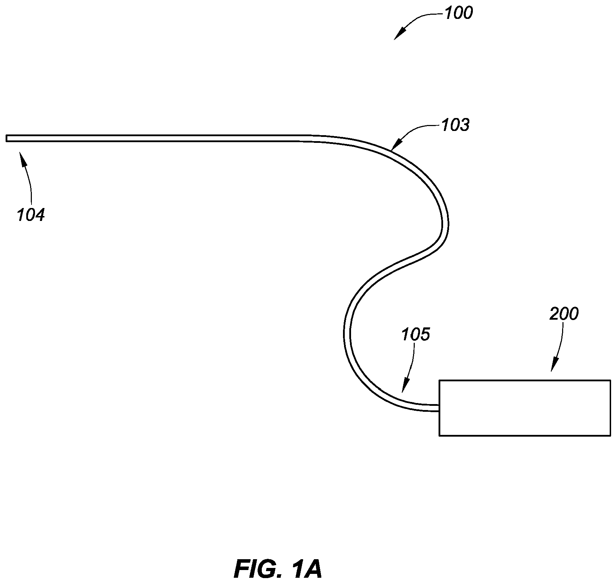

FIG. 1A is a block diagram depicting an example embodiment of an implant delivery system 100 having an elongate delivery device 103 coupled with a proximal control device 200. Here, delivery device 103 is configured as a cystoscope and has a distal end region 104 adapted to be inserted into the patient's urethra (or other lumen or body cavity of the patient) through the urethral orifice. Distal end region 104 preferably has an atraumatic configuration (e.g., relatively soft and rounded) to minimize irritation or trauma to the patient. Elongate delivery device 103 carries or houses one or more implants 102 (not shown) to be delivered or deployed within or adjacent to the prostatic urethra. A proximal end region 105 of delivery device 103 is coupled with proximal control device 200, which remains outside of the patient's body and is configured to be used by the physician or other healthcare professional to control the delivery of one or more implants 102.

### Example Embodiments of Delivery Devices and Related Methods

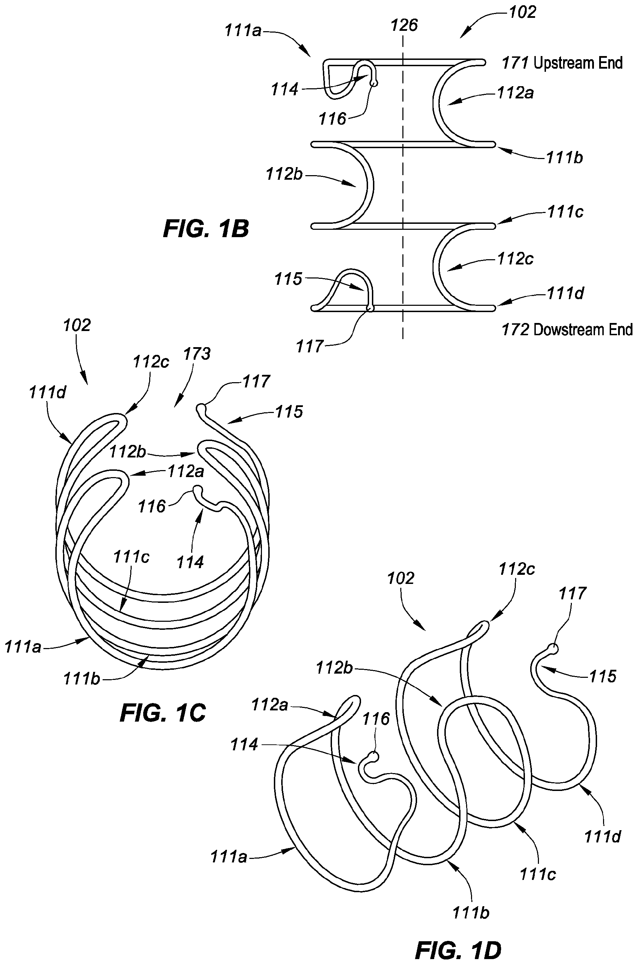

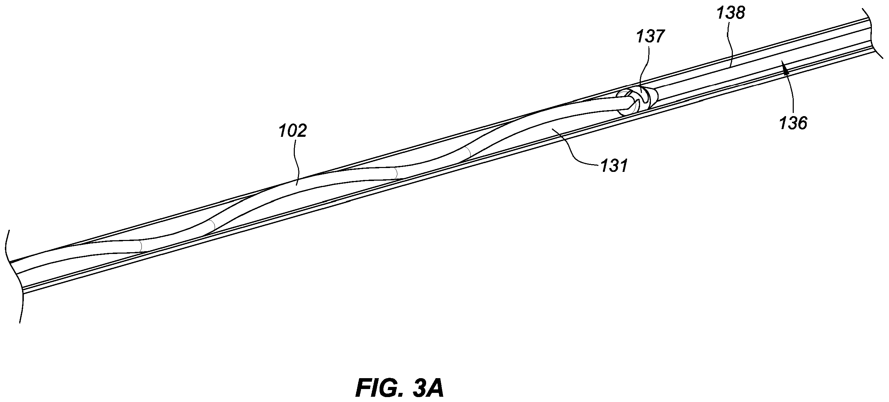

FIGs. 1B, 1C, and 1D are side, end perspective, and side perspective views, respectively, depicting an example embodiment of implant 102 in an at-rest configuration. Implantable device 102 is biased towards the at-rest configuration depicted here and is deformable between the at-rest configuration and a relatively more elongate housed (or delivery) configuration (e.g., see FIG. 3A) for housing implant 102 within delivery device 103.

Implant 102 can be placed in a housed or deployment configuration that is a straight, substantially straight or lineated state with minimal curvature. The term "lineated" refers to an elongate, straight or substantially straight configuration such as would be exhibited upon elongating an implant having a body formed from only one wire-like member for placement with a close fit inside a lumen of a delivery device. Alternatively, implant 102 may be maintained in a crimped (e.g., coil-like) form prior to deployment or in some other configuration.

The at-rest configuration has a relatively greater lateral width, and a relatively shorter longitudinal length than the housed configuration. Upon exiting an open end of delivery device 103, implant 102 is free to laterally expand and longitudinally contract to transition its shape back towards that of the at-rest configuration although restraints imparted by the patient's urethral wall may prevent implant 102 from fully reaching the at-rest configuration. Because implant 102 is elastic and biased towards the at-rest configuration, implant 102 is configured to automatically expand when freed from the restraint of delivery device 103, and can be referred to as "self-expanding." The shape of implant 102 in its deployed state within, e.g., the patient's urethra, can be referred to as the deployed configuration, and will often be a shape that is deformed from the at-rest configuration by the surrounding tissue, although the deployed configuration can be the same as the at-rest configuration.

Implant 102 can be configured in numerous different ways, including any and all of those implant configurations described in U.S. Patent Publ. No. 2015/0257908 ("Indwelling Body Lumen Expander") and/or Int'l Publ. No. WO 2017/184887 ("Systems and Methods for Implants and Deployment Devices").

Implant 102 has a first end 171 and a second end 172. Preferably, first end 171 is positioned closer to the bladder than end 172, and thus, with reference to normal urine flow, end 171 can be referred to as upstream or distal end 171 and end 172 can be referred to as downstream or proximal end 172, although such orientation can be reversed in some embodiments. Implant 102 is formed from one or more discrete bodies (e.g., wires, ribbons, tubular members) of varying geometries. Referring to the embodiment of FIGs. 1B-1D, implant 102 has a main body formed of only one single wire member set in a predetermined coil or coil-like shape. Implant 102 can have two or more expander sections that abut the tissue wall and expand the lumen to maintain patency. In many embodiments, these expander sections are configured as ring-shaped structures 111. In the embodiment of FIGs. 1B-1D there are four ring-shaped structures: 111a, 111b, 111c, and 111d.

Each expander section can be coupled with one or more interconnections 112, such that at least one interconnection 112 extends between each pair of adjacent expander sections. In some embodiments only one interconnection 112 extends between each pair of adjacent expander sections, while in other embodiments two, three, or more interconnections 112 can be present between a pair of adjacent expander sections. In the embodiment of FIGs. 1B-1D there is one interconnection 112 between each adjacent pair of structures 112 for a total of three: 112a, 112b, and 112c. Each interconnection 112 can extend from one ring-shaped structure 111 to an immediately adjacent ring-shaped structure 111. Each interconnection 112 can have a relatively straight shape (not shown) or a curved (e.g., semi-circular or semi-elliptical) shape as shown in FIGs. 1B-1D. Interconnections 112 can also be referred to as "spines."

Some embodiments of implant 102 can therefore include only two ring-shaped structures 111 with only one interconnection 112, only three ring-shaped structures 111 with only two interconnections 112, only four ring-shaped structures 111 with only three interconnections 112, only five ring-shaped structures 111 with only four interconnections 112, only six ring-shaped structures 111 with only five interconnections 112, only seven ring-shaped structures 111 with only six interconnections 112, only eight ring-shaped structures 111 with only seven interconnections 112, only nine ring-shaped structures 111 with only eight interconnections 112, only ten ring-shaped structures 111 with only nine interconnections 112, only eleven ring-shaped structures 111 with only ten interconnections 112, only twelve ring-shaped structures 111 with only eleven interconnections 112, and so on.

Ring-shaped structures 111 are configured to maintain the urethra in a fully or partially open state when expanded from the housed configuration. Device 100 can be manufactured in various sizes as desired, such that the width (e.g., diameter) of each ring-shaped structure 111 is slightly larger than the width of the urethra to "tent" open the urethra and maintain radial fixation force against the prostate. The length of each interconnection 112 determines the spacing between ring-shaped structures 111. As the prostatic urethra can vary in expanded diameter along its length, the various ring-shaped structures 111 can have the same or different widths. For example, in the embodiment depicted here, upstream ring-shaped structure 111a has a relatively smaller width than downstream structures 111b-111d, which have the same width. This can accommodate prostatic urethras that converge to a smaller geometry before the bladder neck.

Each ring-shaped structure 111 can be located or lie in a single plane, and in some embodiments that single plane can be oriented with a normal axis parallel to a central longitudinal axis 126 of implant 102 (as depicted in FIG. 1B). In other embodiments, ring-shaped structures 111 can be located in multiple planes. Ring-shaped structures 111 can extend around central axis 126 to form a complete circle (e.g., a 360 degree revolution) or can form less than a complete circle (e.g., less than 360 degrees) as shown here. Axis 126 is also parallel to the direction of urine flow. Although not limited to such, in many embodiments ring-shaped structures 111 extend between 270 and 360 degrees.

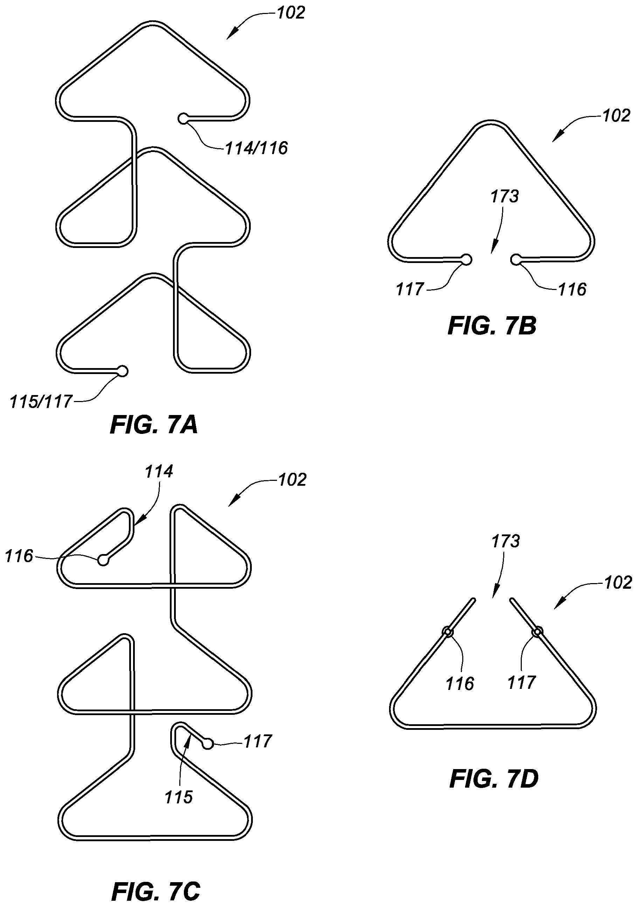

As can be seen from FIGs. 1B-1D, the geometry of implant 102 can have a cylindrical or substantially cylindrical outline shape with a circular or elliptical cross-section. The circle or ellipse can be full (360 degree coverage) or partial (less than 360 degree coverage) as will be described herein. Here, implant 102 is a partial circle with an open side 173 (FIG. 1C). In other embodiments described herein (e.g., FIGs. 7A-8B), implant 102 can have other non-circular or non-elliptical cross-sectional shapes.

Implant 102 can also include a distal engagement member 114 and/or a proximal engagement member 115 that are each configured to engage with elements of delivery device 103. Engagement with delivery device 103 can serve one or more purposes such as allowing control of the release of implant 102, allowing movement of the ends of implant 102 relative to each other, and/or allowing retrieval of implant 102 after deployment, e.g., in an instance where the physician desires to recapture implant 102 and redeploy implant 102 in a different position. In this embodiment, distal engagement member 114 is a wire-like extension from ring-shaped structure 111a that has a curved (e.g., S-like) shape for positioning an atraumatic end 116 (e.g., rounded, spherical, ball-like) in a location suitable for engagement with delivery device 103 and thereby allow control of the distal end region of implant 102. Likewise, proximal engagement member 115 has a curved shape for positioning another atraumatic end 117 in a location suitable for engagement with delivery device 103 and thereby allow control of the proximal end region of implant 102. In other embodiments, distal engagement member 114 and proximal engagement member 115 can be omitted, and delivery device 103 can couple with implant 102 at one or more other distal and/or proximal locations, such as on a ring-shaped structure 111 or interconnect 112. Engagement members 114 and 115 can be formed from a wire or ribbon, etc. having the same or a smaller diameter or width than the wire or ribbon, etc. forming the main body of implant 102. In some embodiments, engagement members 114 and 115 are referred to as "tails" if they extend from ring-shaped structure 111.

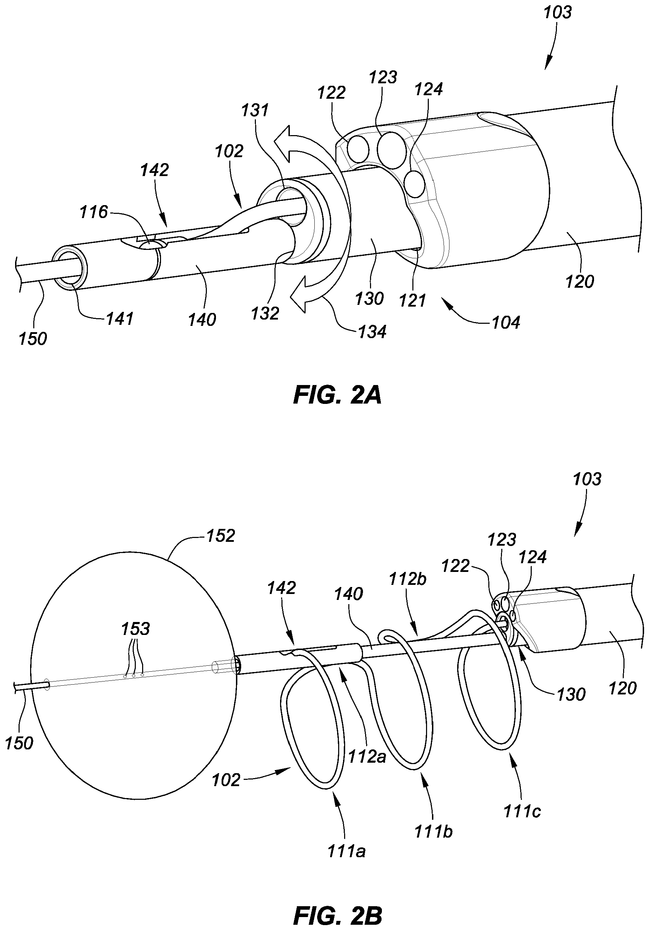

Turning now to implantation, delivery device 103 can include one or more elongate flexible members (e.g., 120, 130, 140, and 150 as described below), each having one or more inner lumens. One or more elongate flexible members of delivery device 103 can be a solid or a non-hollow member with no inner lumen. FIG. 2A is a perspective view depicting an example embodiment of distal end region 104 of a delivery device 103. In this embodiment, delivery device 103 includes a first elongate tubular member 120, a second elongate tubular member 130, a third elongate tubular member 140, and a fourth elongate tubular member 150. Delivery device 103 can vary and in other embodiments can include more or less tubular members.

In this embodiment, first elongate tubular member 120 is the outermost tubular member and is flexible yet provides support for members contained therein. First tubular member 120 is referred to herein as outer shaft 120 and can have one or more inner lumens. In this embodiment, outer shaft 120 includes a first inner lumen 121 housing second elongate tubular member 130, which is referred to herein as inner shaft 130. Outer shaft 120 and inner shaft 130 are each controllable independent of the other. Inner shaft 130 can slide distally and proximally within lumen 121 and is shown here partially extending from an open distal terminus of outer shaft 120.

Outer shaft 120 has a proximal end (not shown) coupled with proximal control device 200. In this embodiment, outer shaft 120 includes three additional lumens 122, 123, and 124. An illumination device (not shown) and an imaging device (not shown) can be housed in two of lumens 122-124 (e.g., lumens 122 and 123). The imaging device can utilize any desired type of imaging modality, such as optical or ultrasound imaging. In one example embodiment the imaging device utilizes a forward (distal) looking CMOS imager. The illumination device can be configured to provide adequate illumination for optical imaging, and in one embodiment includes one or more light emitting diodes (LEDs). In embodiments where illumination is not required, such as for ultrasound imaging, the illumination device and its respective lumen can be omitted. The illumination device and/or the imaging device can each be fixedly secured at the distal terminuses of lumens 122 and 123, or each can be slidable within lumens 122 and 123 to allow advancement further distally from outer shaft 120 and/or retraction into outer shaft 120. In one example embodiment, the illumination device and the imaging device are mounted together and only a single lumen 122 or 123 is present for that purpose. The remaining lumen (e.g., lumen 124) can be configured as an irrigation or flush port from which fluid such as saline can be introduced to the urethra to flush the region and provide adequate fluid through which implant 102 and the surrounding prostatic urethra wall can be imaged.

Inner shaft 130 can include one or more inner lumens for housing one or more implants 102 and/or other components. In this embodiment, inner shaft 130 includes a first lumen 131 in which one or more implants 102 can be housed, and a second lumen 132 in which third elongate tubular member 140 can be housed. In this embodiment, third elongate tubular member 140 is configured to releasably couple with the distal end region of implant 102 and is referred to as a distal control member or tether 140. Distal control member 140 can be slidably advanced and/or retracted with respect to inner shaft 130. Distal control member 140 can include an inner lumen 141 that houses fourth elongate tubular member 150, which is shown here extending from an open distal terminus of distal control member 140. Fourth elongate tubular member 150 is configured to anchor delivery device 103 with respect to the patient's anatomy, e.g., to keep components of delivery device 103 stationary with respect to the anatomy during deployment of implant 102, and is referred to as anchor delivery member 150.

In the configuration depicted in FIG. 2A, anchor delivery member 150 is extended from lumen 141 of distal control member 140, and distal control member 140 along with inner shaft 130 are shown extended from lumen 121 of outer shaft 120. When delivery device 130 is advanced through the urethra, anchor delivery member 150 is preferably housed entirely within distal control member 140, and distal control member 140 along with inner shaft 130 are retracted from the positions shown in FIG. 2A such that they reside within lumen 121 of outer shaft 120 and do not extend from the open distal terminus of lumen 120. In other words, in some embodiments the open distal terminus of outer shaft 120 forms the distalmost structure of device 103 upon initial advancement through the urethra. This facilitates steering of delivery device 103 by outer shaft 120. The physician can advance distal end region 104 of delivery device 103 to be in proximity with the desired implantation site, or entirely into the patient's bladder. Anchor delivery member 150 can be exposed from the open distal terminus of distal control member 140, either by distally advancing anchor delivery member 150 further into the bladder, or if already present within the bladder, then by proximally retracting the other components of delivery device 103. At this point the anchor from anchor delivery member 150 can be deployed in the bladder.

The placement of these components within system 100 is not limited to the embodiments described with respect to FIG. 2A. In some embodiments, outer shaft 120 can be omitted altogether. In such embodiments, visualization of the deployment procedure can be accomplished with external imaging such as fluoroscopy, where implant 102 and delivery device 103 can be radiopaque or can include radiopaque markers, and where the imaging and illumination lumens 122 and 123 (and the imaging and illumination devices), as well as the irrigation lumen are omitted. In some embodiments, instead of distal control member 140 being slidably received within inner shaft 130, distal control member 140 can be slidable within a lumen of outer shaft 120 (either the same lumen receiving inner shaft 130 or a different lumen). Similarly, instead of anchor delivery member 150 being slidably received within distal control member 140, anchor delivery member 150 can be slidable within a lumen of outer shaft 120 (either the same lumen receiving inner shaft 130 and/or anchor delivery member 150 or a different lumen) or a lumen of inner shaft 130 (either the same lumen receiving distal control member 140 or a different lumen). In some embodiments, outer shaft 130 has a separate and distinct lumen for each of members 130, 140, and 150, and can be configured to deploy implant 102 around members 140 and 150.

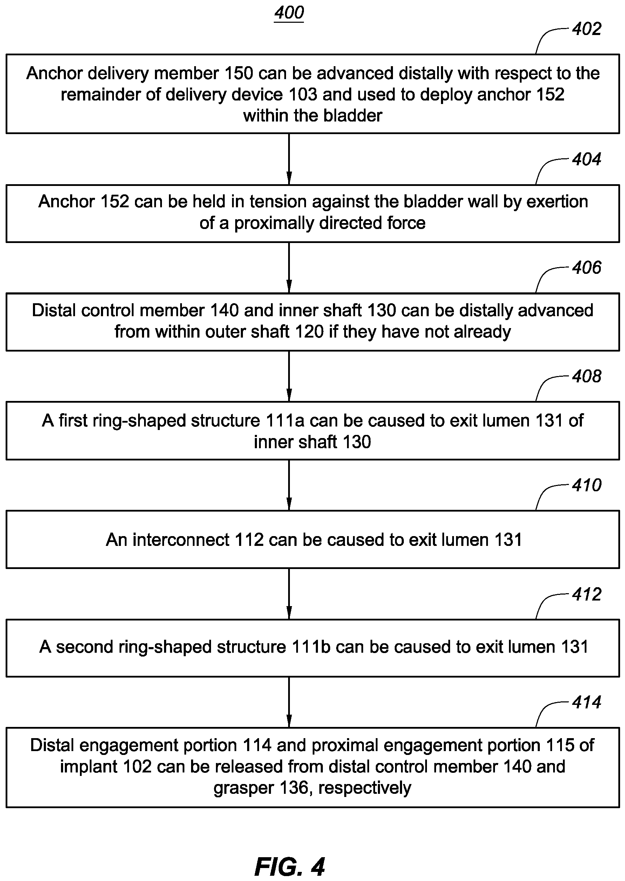

FIG. 2B is a perspective view depicting distal end region 104 of delivery device 103 with the various components deployed. In this embodiment, anchor delivery member 150 includes an anchor 152 in the form of an inflatable member or balloon. Other embodiments of anchors 152 are described with respect to FIGs. 4A-4G. Anchor 152 expands (or otherwise transitions) to a size greater than that of the bladder neck such that anchor 152 resists proximal retraction (e.g., a relatively light tension). In embodiments where anchor 152 is a balloon, that balloon can be an elastic or inelastic and inflatable with an inflation medium (e.g., air or liquid such as saline) introduced into balloon 152 through one or more inflation ports 153. Here three inflation ports 153 are located on the shaft of anchor delivery member 150 and communicate with an inflation lumen that extends proximally back to proximal control device 200, which can include a port for inflation with a syringe. Upon deployment of anchor 152, the physician can proximally retract delivery system 100 until anchor 152 is in contact with the bladder neck and/or wall (if not already).

The physician can use the imaging device of outer shaft 120 to move delivery device 103 proximally away from anchor 152 until the physician is in the desired position within the urethra to begin deployment of implant 102. A retainer 142 on distal control member 140 is releasably coupled with distal engagement member 114 of implant 102. The physician can position retainer 142 in a location along the length of the urethra where the physician desires the distal end of implant 102 to deploy. This can involve moving distal control member 140 and inner shaft 130, together, proximally and/or distally with respect to anchor delivery member 150. In another embodiment, the position of retainer 142 is fixed with respect to anchor 152 such that the longitudinal position of implant 102 within the anatomy is set by the system independently of any manipulation by the physician. The coupling of distal engagement member 114 with retainer 142 also permits the physician to manipulate the radial orientation of implant 102 by rotating distal control member 140 and inner shaft 130 together. Active or passive shaping of distal control member 140 may allow for a more desirable placement of implant 102. For example, member 140 may have a curvature that places the implant in a more anterior anatomical position. This curvature may be inherently set in member 150 or actively applied by the physician though a separate entity such as a control wire. Once in the desired location and orientation, the physician can proximally retract inner shaft 130 with respect to distal control member 140 to initiate deployment of implant 102.

Distal engagement member 114 is held in place with respect to distal control member 140 by retainer 142, and proximal retraction of inner shaft 130 with respect to distal control member 140 causes ring-shaped structures 111 to begin to deploy in sequence (111a, then 111b, then 111c, then 111d (not shown)). Distal control member 140 can remain stationary or be moved longitudinally with respect to the urethra during deployment. Distal control member 140 can be significantly flexible to passively accommodate tortuous anatomy. In some embodiments, distal control member 140 has a predefined curve to assist in navigation.

To assist in deployment, inner shaft 130 can rotate clockwise and counterclockwise (as depicted by arrow 134) about distal control member 140. Referring back to FIGs. 1B-1C, implant 102 has a non-constant direction of winding that, when viewed as commencing at distal engagement member 114, proceeds clockwise along ring-shaped structure 111a, then reverses along interconnect 112a to a counterclockwise direction for ring-shaped structure 111b, then reverses along interconnect 112b to a clockwise direction for ring-shaped structure 111c, and then reverses along interconnect 112c to a counterclockwise direction for ring-shaped structure 111d, until ending at proximal engagement member 115. Depending on the direction of winding of the portion of implant 102 about to exit the open distal terminus of lumen 131, the transition of implant 102 towards the at-rest configuration can impart a torque on shaft 130 if shaft 130 is not actively rotated as implant 102 is deployed. That torque can cause shaft 130 to passively rotate (without user intervention) either clockwise or counterclockwise accordingly. In certain embodiments described elsewhere herein, shaft 130 is actively rotated during deployment. Rotation of inner shaft 130 with respect to distal control member 140 thus allows delivery device 103 to rotate and follow the direction of winding of implant 102. In some embodiments, all ring-shaped structures 111 are wound in the same direction, clockwise or counterclockwise (e.g., as in the case of a fully spiral or helical implant), or do not have a set direction of winding.

In this or other embodiments, the distal end region of inner shaft 130 is configured to be relatively more flexible than the more proximal portion of inner shaft 130, which can permit avoidance of excessive motion of the rest of device 103 during deployment, resulting in better visualization and less tissue contact by device 103. Such a configuration can also reduce the stress imparted on implant 102 by device 103 during delivery. For example, the portion of inner shaft 130 extending from outer shaft 120 during deployment can be relatively more flexible than the portion of inner shaft 130 that remains within outer shaft 120, thus allowing inner shaft 130 to flex more readily as implant 102 exits inner lumen 131. This in turn can stabilize delivery device 103 and allow the physician to obtain stable images of the appointment process.

FIG. 2B depicts implant 102 after three ring-shaped structures 111a, 111b, and 111c have been deployed. Proximal retraction of shaft 130 continues until the entirety of implant 102, or at least all of ring-shaped structures 111, have exited lumen 131. If the physician is satisfied with the deployed position of implant 102 and the deployed shape of implant 102, then implant 102 can be released from delivery device 103.

Release of the distal end of implant 102 can be accomplished by releasing retainer 142. Retainer 142 can be a cylindrical structure or other sleeve that linearly or rotationally actuates over a cavity or recess in which a portion of implant 102 is housed. In the embodiment of FIG. 2B, retainer 142 includes an opening or slot that allows distal engagement member 114 to pass therethrough. Retainer 142 can rotate with respect to the cavity or recess in which distal engagement member 114 (not shown) is housed until the opening or slot is positioned over member 114, at which point member 114 is free to release from distal control member 130. Rotation of retainer 142 can be accomplished by rotation of a rotatable shaft, rod or other member coupled with retainer 142 (and accessible at proximal control device 200).

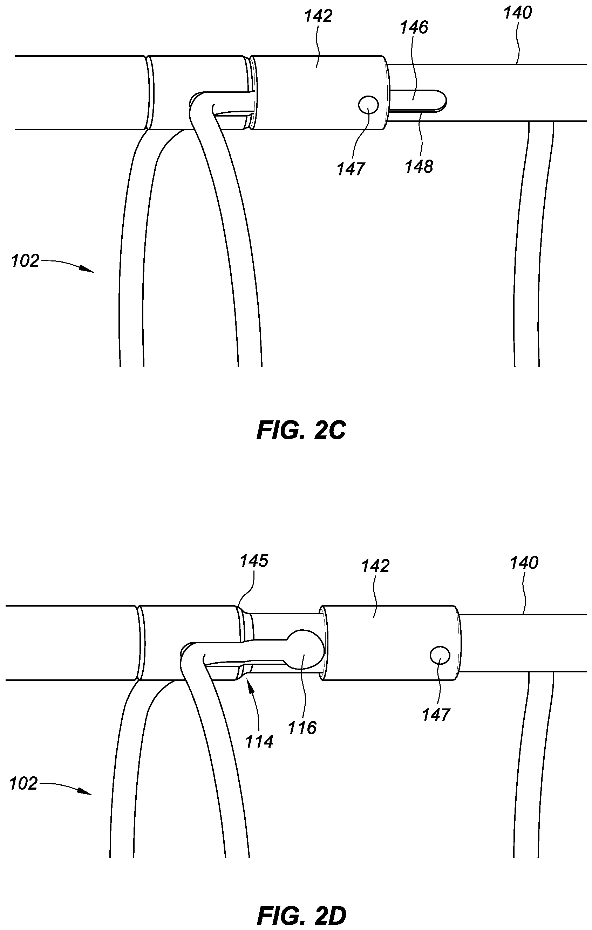

FIGs. 2C and 2D are perspective views depicting another example embodiment of system 100 with a different embodiment of retainer 142 shown in more detail. Here, retainer 142 slides distally and/or proximally with respect to distal control member 140. Distal engagement member 114 of implant 102 can be received within a corresponding recess of distal control member 140. Retainer 142 can slide over distal engagement member 114 while received within this recess until retainer 142 abuts a stepped portion of member 140. A control wire 146 extends within the length of control member 140, either in the same lumen as anchor delivery member 150 or in a different lumen. Control wire 146 couples with retainer 142 with an enlarged portion 147 from which control wire 146 can be routed into member 140 through an opening 148.

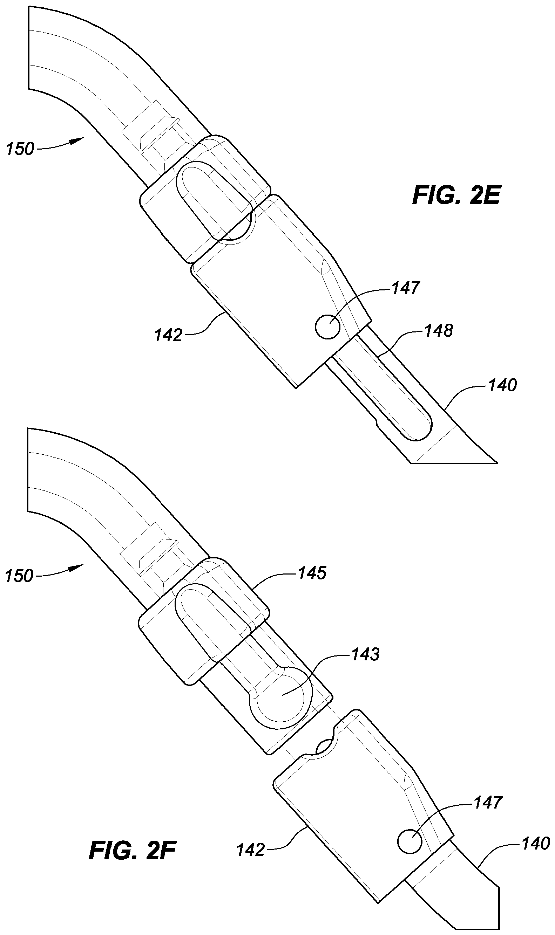

Engagement member 114 can be placed within the recess and retainer 142 can be advanced over engagement 114 to secure the distal end of implant 102 to control member 140. Upon satisfactory deployment of implant 102 within the urethra, e.g., in the state of FIG. 2C, retainer 142 can be proximally retracted with control wire 146 to expose engagement member 114 and permit its release from member 140. FIGs. 2E and 2F are perspective views depicting another embodiment of system 100 with another configuration for retainer 142 that operates in similar fashion to that described with respect to FIGs. 2C and 2D. Here, implant 102 is not shown and recess 143 in which distal engagement member 114 can be received is shown in more detail.

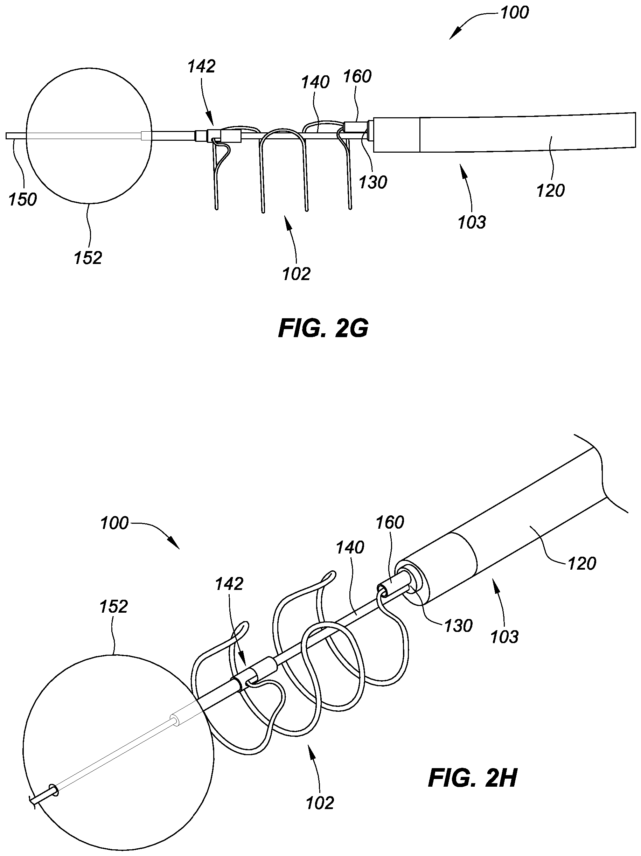

FIGs. 2G and 2H are side and perspective views, respectively, of another example embodiment of system 100. In this embodiment, inner shaft 130 includes a flexible distal extension 160 in which is located inner lumen 13 1 (not shown). In this configuration, the open distal terminus of lumen 131 is located distal to the open distal terminus of lumen 132 (not shown) from which distal control member 140 extends. Lumens 122, 123, and 124 (not shown) are located on outer shaft 120 opposite to distal extension 160. Flexible distal extension 160 contributes to the flexibility to stabilize the delivery system, as well as to stabilize the image. Flexible extension 160 helps align ring-shaped structures 111 in a planar manner, and helps vector implant 102 (e.g., point radially) toward the urethral wall during deployment.

Release of the proximal end of implant 102 is also controllable. FIG. 3A is a partial cross-sectional view depicting an example embodiment of system 100 with a portion of implant 102 shown within inner lumen 131 of inner shaft 130. Here, implant 102 is in the lineated state prior to deployment with proximal engagement member 115 coupled with a grasper 136 that is slidable distally and/or proximally within lumen 131. Grasper 136 can include a distal end region 137 on or coupled with a shaft 138. Grasper 136 is preferably controllable to rotate and longitudinally translate (e.g., push and pull) implant 102 with respect to inner shaft 130.

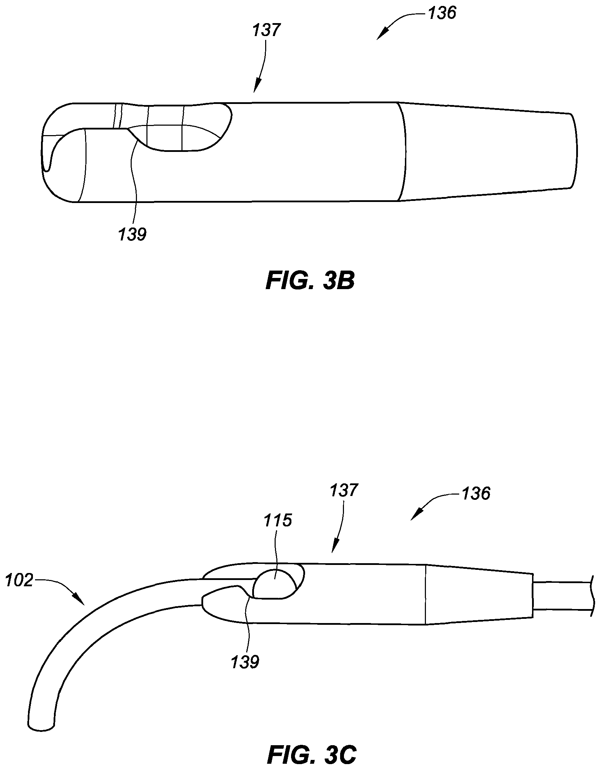

FIGs. 3B and 3C are perspective views depicting an example embodiment of distal end region 137 of grasper 136 without implant 102 and with implant 102, respectively. Grasper 136 includes a recess (also referred to as a cavity or pocket) 139 for receiving and holding proximal engagement member 115. Here, the enlarged portion 117 is retained within recess 139 by a distal necked down region having a relatively smaller width. While within inner lumen 131, the sidewalls of inner shaft 130 maintain proximal engagement member 115 within recess 139. When distal end region 137 exits inner lumen 131 (either by retracting inner shaft 130 with respect to grasper 136 or by advancing grasper 136 with respect to inner shaft 130), the restraint imparted by the inner shaft sidewalls is no longer present and engagement member 115 is free to release from grasper 136. Thus, when the physician is satisfied with placement of the deployed implant 102, distal engagement member 114 can be released by moving retainer 142 and permitting distal engagement member 114 to decouple from control member 140, and proximal engagement member 115 can be released by exposing grasper 136 from within inner shaft 130 and permitting proximal engagement member 115 to decouple from grasper 136.

Grasper 136 can also assist in loading implant 102. In some embodiments, application of a tensile force on implant 102 with grasper 136 (while the opposite end of implant 102 is secured, for example, by retainer 142) facilitates the transition of implant 102 from the at-rest configuration to a lineated configuration suitable for insertion of implant 102 into inner shaft 130.

Anchor delivery member 150 can have multiple different configurations and geometries (e.g., including those that extend in one direction across the bladder wall, two directions across the bladder wall (e.g., left and right), or three or more directions across the bladder wall). Additional example embodiments of anchor delivery members are described in Int'l Publ. No. WO 2018/107123 ("Systems, Devices, and Methods for the Accurate Deployment of an Implant in the Prostatic Urethra"). Embodiments of proximal control devices 200 suitable for use with the present embodiments are also described in the incorporated Int'l Publ. No. WO 2018/107123. Additional embodiments of delivery devices usable with the embodiments of implant 102 described herein are also described in the incorporated U.S. Patent Publ. No. 2015/0257908 and Int'l Publ. No. WO 2017/184887 references.

### Example Embodiments of Delivery Methods

FIG. 4 is a flow diagram depicting an example embodiment of a method 400 of delivering implant 102 using system 100. Distal end region of outer shaft 120 is inserted into the urethra, preferably with inner shaft 130, distal control member 140, and anchor delivery member 150 in retracted states fully contained within outer shaft 120 such that no part is extending from the open distal terminus of outer shaft 120. After advancement into the urethra, at step 402 anchor delivery member 150 is advanced distally with respect to the remainder of delivery device 103 (e.g., members 120, 130, and 140) and used to deploy anchor 152 within the bladder. In some embodiments, deployment of anchor 152 can be the inflation of one or more balloons (e.g., as depicted in FIG. 2B) by the introduction of an inflation medium through an injection (e.g., luer taper) port. In other embodiments deployment of anchor 152 can be the advancement of one or more wire-form members from anchor delivery member 150 such that they deflect into a position that opposes the bladder wall (e.g., as described in Int'l Publ. No. WO 2018/107123). The longitudinal positioning (e.g., advancement and retraction) of anchor delivery member 150 and/or any wire-form members can be accomplished manually by the user manipulating a proximal end of anchor delivery member 150 and/or any wire-form members either directly or with proximal control device 200.

At step 404, anchor 152 can be held in tension against the bladder wall by exertion of a proximally directed force on device 200. Anchor 152 can therefore provide an ordinate for system 100 from which to deploy implant 102 in an accurate location. This feature can ensure the implant is not placed too close to the bladder neck.

At 406, distal control member 140 and inner shaft 130 can then be distally advanced from within outer shaft 120 if they have not already (for example, step 406 can occur prior to steps 402 and/or 404). The user can manipulate the position of proximal control device 200 with the aid of imaging (as described herein) until implant 102 is in the desired position. Once implant 102 is in the desired position, the implant deployment procedure can begin. The steps for implant deployment can be performed automatically by user actuation of proximal control device 200, or the steps can be performed directly by hand manipulation of each component of delivery device 103, or by a combination of the two as desired for the particular implementation.

In some embodiments, deployment of implant 102 from within lumen 131 is fully accomplished by (1) distally advancing grasper 136 with respect to inner shaft 130, while inner shaft 130 is not moved, while in other embodiments, deployment of implant 102 from within inner lumen 131 is fully accomplished by (2) proximally retracting inner shaft 130 with respect to grasper 136 while grasper 136 is not moved. In some embodiments, deployment of implant 102 is fully accomplished by (3) a combination of both movements. In still other embodiments, deployment of implant 102 is fully accomplished by (1), (2), or (3) in combination with one or more rotations of inner shaft 130, in one or more directions (e.g., clockwise or counterclockwise) with respect to distal control member 140.

### Example Embodiments of Implant Placement

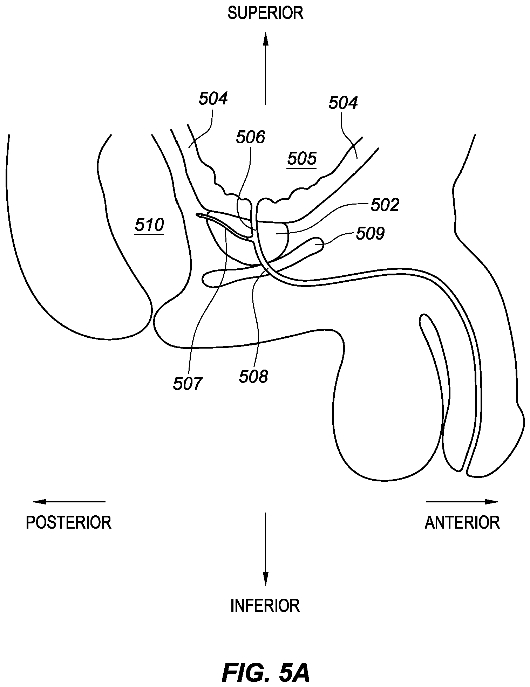

All the embodiments of system 100 described herein can be used to deliver implant 102 to various locations in proximity to the prostate gland, or other locations within the human anatomy. FIG. 5A is a cross-section of the male anatomy that provides context for use in describing various examples of implantation locations within the prostatic urethra. Here, prostate gland 502 is centrally located with bladder wall 504 and bladder 505 located superiorly. The prostatic urethra 506 extends inferiorly from bladder 505 past ejaculatory duct 507 and through prostate gland 502. The prostatic urethra 506 becomes the membranous urethra 508 at the general location of the external urethral sphincter 509 and continues on to exit the body. The rectum is indicated by 510.

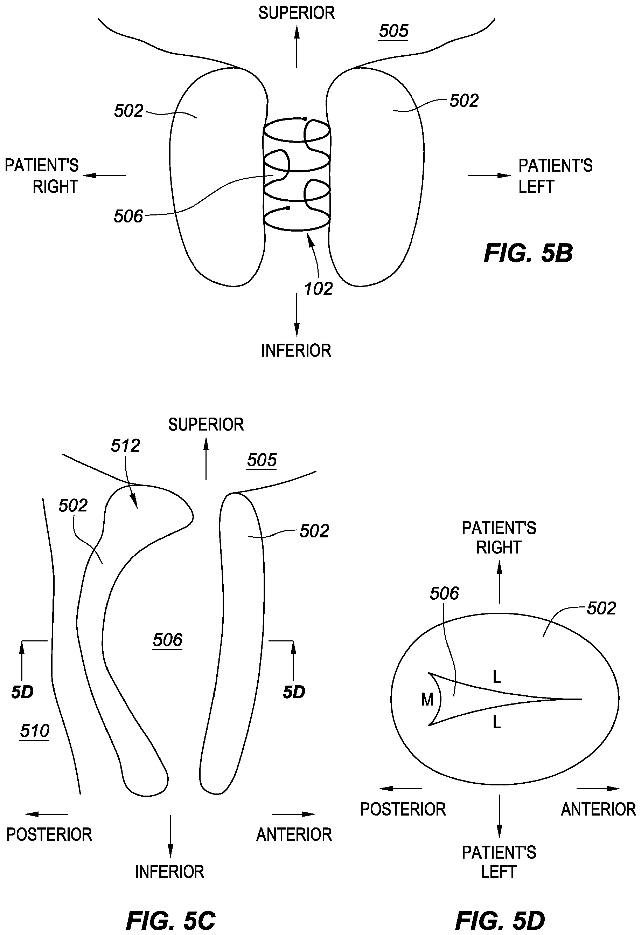

FIG. 5B is a cross-section rotated from the viewpoint of FIG. 5A such that the posterior direction extends into the page in the anterior direction extends out of the page. Here an example embodiment of implant 102 is shown positioned within prostatic urethra 506. Implant 102 is generally positioned centrally within prostatic urethra 506 as viewed from this perspective, in other words, generally an equal distance from the superior and inferior edges of prostate gland 502. Placement of implant 102 is generally at the discretion of the medical professional and can be offset either superiorly or inferiorly from the positions shown here, however a position within the prostatic urethra 506 is generally preferred.

FIG. 5C is a sagittal cross-section depicting the area of prostate gland 502 from generally the same perspective as that of FIG. 5A, but with more detail. Here, prostate gland 502 is in an enlarged state with a median lobe 512 that protrudes into prostatic urethra 506. FIG. 5D is a cross-section taken along line 5D-5D of FIG. 5C. This cross-section is a plane perpendicular to the longitudinal axis of the urethra and shows the slit-like nature of prostatic urethra 506 in this enlarged prostate gland 502 where the width of urethra 506 widens as it progresses from the anterior to the posterior side. Lateral lobes are indicated with "L" and the median lobe is indicated with "M."

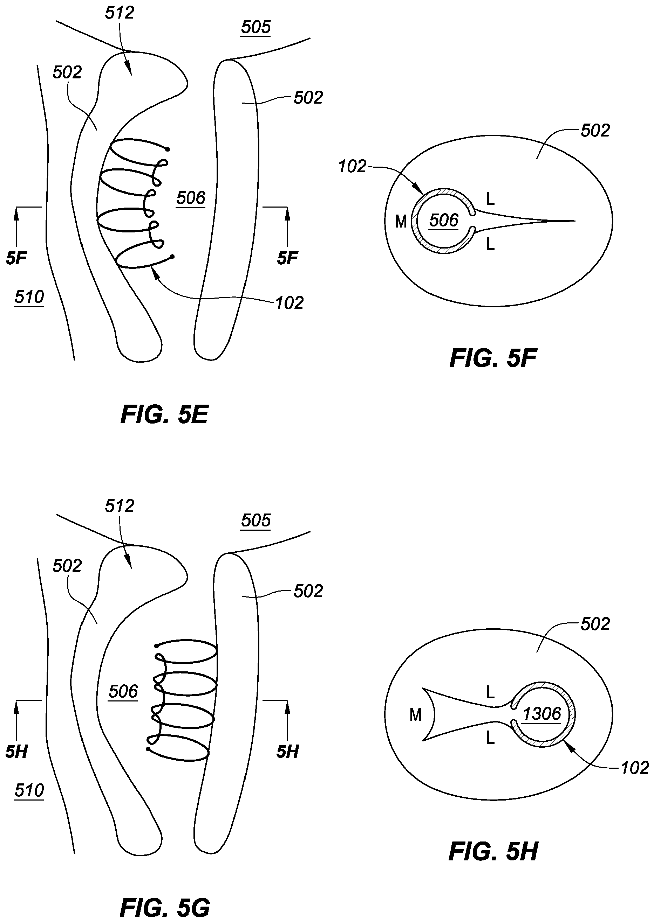

FIG. 5E depicts an example embodiment of a posteriorly placed implant 102 within the example anatomy described with respect to FIG. 5C and FIG. 5F is a cross-section taken along line 5F-5F of FIG. 5E. As can be seen here, implant 102 is placed generally along the posterior most surface of the prostatic urethra 506. Implant 102 is sized to have a maximum diameter that is less than the width of prostatic urethra 506 at its maximum central width (e.g., less than 50% of the width, less than 65% of the width, less than 80% of the width, etc.) such that implant 102 can be described as residing substantially on the posterior side of prostatic urethra 506, and not in contact with the anterior most side of urethra 506. The implications of this placement are shown in FIG. 5F where the opening through prostate gland 502 that is created by implant 102 is positioned primarily on the posterior side of prostate gland 502 and urethra 506. Implant 102 can be instilled with a bias towards a predetermined bend, such as depicted here, e.g., by heat-setting during the manufacturing process. The bend can facilitate placement posteriorly as shown here or anteriorly as shown below with respect to FIG. 5G.

FIG. 5G depicts an example embodiment of an anteriorly placed implant 102 within the example anatomy described with respect to FIG. 5C and FIG. 5H is a cross-section taken along line 5H-5H of FIG. 5G. As can be seen here, implant 102 is placed generally along the anterior most surface of prostatic urethra 506. Implant 102 can be sized to have a maximum diameter that is less than the width of prostatic urethra 506 at its maximum central width (e.g., less than 50% of the width, less than 65% of the width, less than 80% of the width, etc.) such that implant 102 can be described as residing substantially on the anterior side of prostatic urethra 506, and not in contact with the posterior most side of urethra 506. The implications of this placement are shown in FIG. 5H where the opening through prostate gland 502 that is created by implant 102 is positioned primarily on the anterior side of prostate gland 502 and urethra 506.

With both the posterior placement and the anterior placement, implant 102 can still be placed generally centrally with respect to prostate gland 502 as shown in FIG. 5B. Deployment of implant 102 in a posterior or anterior position is generally at the discretion of the medical professional. Other variations of placement can also be used including placements that are centrally located between the posterior most side and interior most side of urethra 506, as well as variations in sizing such that implant 102 has a relatively larger or smaller diameter with respect to prostate 502 than shown here.

### Additional Example Embodiments of Implants

Additional embodiments of implant 102 are now described. Unless stated otherwise, each of the following embodiments described in this section are variants of the embodiments of implant 102 already described, and thus all features and variations of implant 102 described thus far are equally applicable to the following embodiments and, for purposes of brevity, will not be repeated.

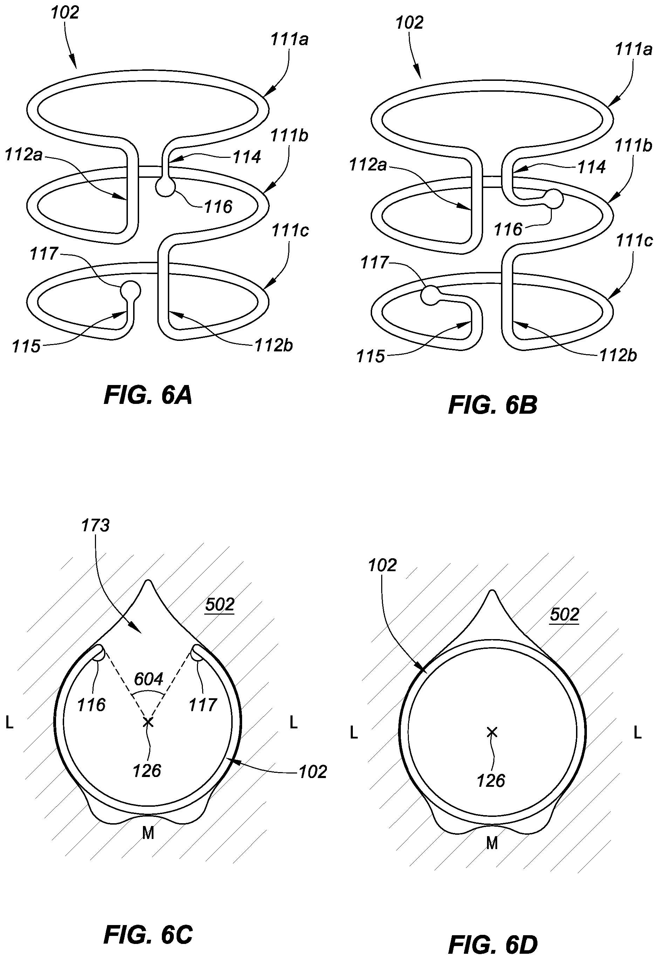

FIGs. 6A and 6B are perspective views depicting additional example embodiments of implant 102. FIG. 6C is an end on view depicting these embodiments within prostate gland 502. Like the embodiment of FIGs. 1B-1D, these embodiments have open side 173 where an angle 604 (FIG. 6C) from one to ninety degrees (or greater) is present between center axis 126 and spine sections 112a and 112b. No portion of the implant body resides in open side 173. The embodiment depicted in FIG. 6C has a radial opening of approximately 60 degrees, while the embodiment depicted in FIG. 5F has a radial opening of approximately 30 degrees. This is in contrast to an implant that has full 360 degree coverage as depicted in FIG. 6D.

An open side implant 102 with radial space between spine sections 112 can serve to reduce urine exposure in the space between the lateral lobes L, prevent implant rotation, and allow for easier passage of a catheter or cystoscope through implant 102. When an open-side implant 102 is deployed into the urethra, the spines 112 can rest against and embed into the lateral lobes L of the prostate 502. In some embodiments, spines 112 lie on alternating sides from one ring-shaped structure 111 to the next (see, e.g., FIGs. 1B-1D, 6A-6B, etc.) such that at least one implant spine section 112 embeds into each lateral lobe L.

In some embodiments, such as depicted in FIG. 5F and 6C, implant 102 can be placed such that the portion of each ring-shaped structure 111 farthest from the spines 112 rests on the posterior surface of the prostate on the urethral crest and spines 112 are aligned along each lateral lobe L on the anterior side of implant 102. In other embodiments, such as depicted in FIG. 5H, implant 102 is aligned 180 degrees from the previously described configuration such that the portion of each ring-shaped structure 111 farthest from spines 112 rests at the anterior extreme of the anterior commissure and spines 112 are aligned along each lateral lobe L on the posterior side of implant 102. In embodiments with less than 360-degree radial coverage, alternating clockwise and counterclockwise orientations from one ring-shaped structure 111 to the next can be desirable, whereas in embodiments with 360-degree coverage, ring orientations that are alternating, constant, or some other combination can be desirable.

In the embodiments described herein, distal engagement member 114 and/or proximal engagement member 115 may taper from the main wire body of implant 102 to a reduced wire diameter to reduce stiffness, improve flexibility, improve compatibility with a small-profile delivery device 103, reduce stress to delivery device 103 before and during implantation, reduce trauma to the urethral tissue during and after implantation, or improve ability to fit within delivery device 103 without undergoing excessive strain. Enlarged ends 116 and 117 can be ball-shaped (or otherwise as described herein) to reduce the risk of tissue trauma. The enlarged ends 116 and 117 can be approximately 1.5 to 2.0 times the diameter of the adjoining wire.

In some embodiments, engagement features 114 and 115 are "tucked" so that they do not extend beyond the plane of the terminal ring-shaped structure 111 of the implant. This minimizes the likelihood that the feature 114 or 115 will puncture tissue or otherwise cause trauma. Furthermore, this feature maximizes the axial length of ring-shaped structures 111, which maximizes the prostatic urethral length the implant can support. In FIG. 6A, distal engagement feature 114 extends proximally from ring-shaped structure 111a, while proximal engagement feature 115 extends distally from ring-shaped structure 111c. In FIG. 6B, distal engagement feature 114 doubles back and extends first proximally and then radially in a direction opposite the direction of winding of ring-shaped structure 111a. Proximal engagement feature 115 doubles back and extends first distally and then radially in a direction opposite the direction of winding of ring-shaped structure 111c.

In some embodiments it is desirable for the distal-most ring-shaped structure 111 to be deployed and chronically maintained to be concentric with the bladder neck opening, e.g., to ensure optimal flow rate improvement. This is generally more important in the anterior-posterior aspect as the lateral aspects tend to center implant 102 by way of the impinging lateral lobes of the prostate. In a lineated implant configuration, the distal engagement feature 114 that dictates axial implant placement may not intersect center axis 126 of the implant and instead exist somewhere along the radial periphery of the implant, which may result in eccentric deployment.

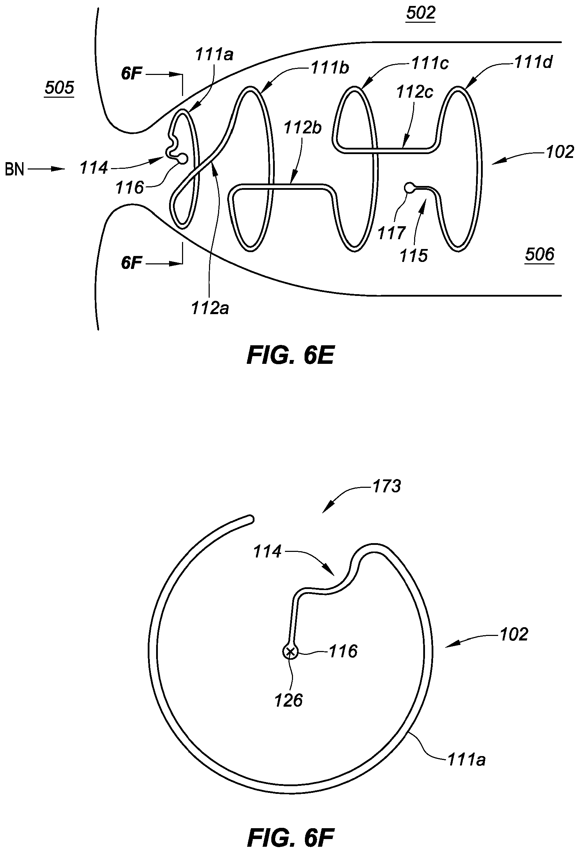

FIGs. 6E and 6F are perspective and end-on views, respectively, depicting an example embodiment of implant 102 with distal engagement feature 114 positioned to assist in concentric placement. Here, implant 102 includes ring-shaped structure 111a with a relatively smaller diameter for placement in proximity with the bladder neck BN. Distal engagement feature 114 extends from the radial periphery of ring shaped-structure 111a into the radial center of implant 102 such that enlarged end 116 is generally in the same position as center axis 126. Distal engagement feature 114 can extend into the radial center with an indirect, curved (S-shaped) path as shown here, to facilitate release, or can extend directly (straight line) in which case the length of feature 114 is approximately one half of the diameter of ring 111a. Delivery device 103 (not shown) may grasp implant 102 at the center of the urethra, which may ensure concentric deployment. In other embodiments, distal engagement feature 114 can be placed on an intermediate ring-shaped structure (e.g., ring-shaped structure 111b), rather than on the distal most 111a. Since the lateral lobes maintain lateral concentricity of the distal-most ring-shaped structure 111a, the delivery system (and its anchoring system) can thus provide anterior-posterior concentricity of implant 102. Because distal-most ring-shaped structure 111a is smaller than the next proximal structure 111b, spine 112a travels both radially and proximally to connect to ring 11 1b at the appropriate radial location to match ring-shaped structures 111c and 11 1d. Instillation of the bias to this embodiment can be performed after the main body wire has been wrapped around a cylindrical portion of a mandrel to form structures 111b, 112b, 111c, 112c, and 111d, and a conical portion of the mandrel to form structures 111a and 112a.

Embodiments of implant 102 can use outward radial force and invagination into the urethral wall to provide fixation within the urethra. Implant 102 can include additional features to improve fixation or rotational stability. These features may also serve to minimize exposure of the implant to urine. Examples thereof include various radial cross-section shapes, anchors, hooks, indentations or protrusions on the surface facing the posterior surface to accommodate the urethral crest and/or verumontanum, or other mechanisms.

The expander sections 111, which in many embodiments are configured as circular or substantially circular ring-shaped sections when viewed end-on (the viewing axis is aligned with center axis 126), can be configured as non-circular ring-shaped sections as well. In all of the embodiments herein, the cross-section can have full 360 degree coverage or partial coverage less than 360-degrees (e.g., with an open side 173).

FIGs. 7A and 7B are perspective and end-on views, respectively, depicting a first example embodiment of implant 102 with triangular ring-shaped structures 111. Here, the triangular ring-shaped structures 111 have rounded corners with open side 173 at the center of the bottom side (FIG. 7B). FIGs. 7C and 7D are perspective and end-on views, respectively, depicting a second example embodiment of implant 102 with triangular ring-shaped structures 111, but with open side 173 where one (top) of the three corners would be positioned, such that two sides of the triangle converge at open side 173. Different degrees of rounded corners for the triangle can be present, including sharp corners with little to no rounding. These embodiments can be positioned in any desired radial orientation (e.g., with a corner positioned anteriorly and the opposite side positioned posteriorly against the median lobe, or vice-versa).

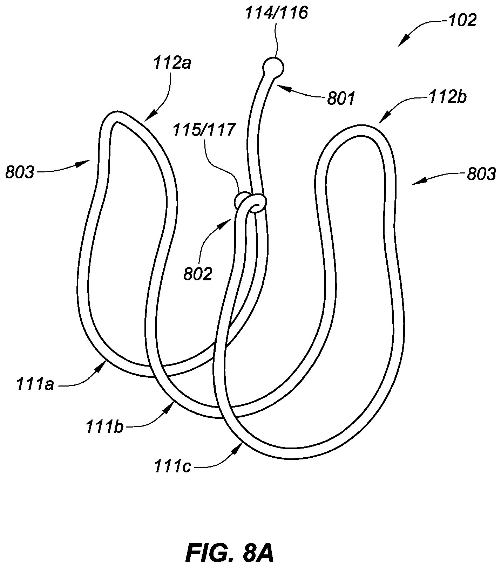

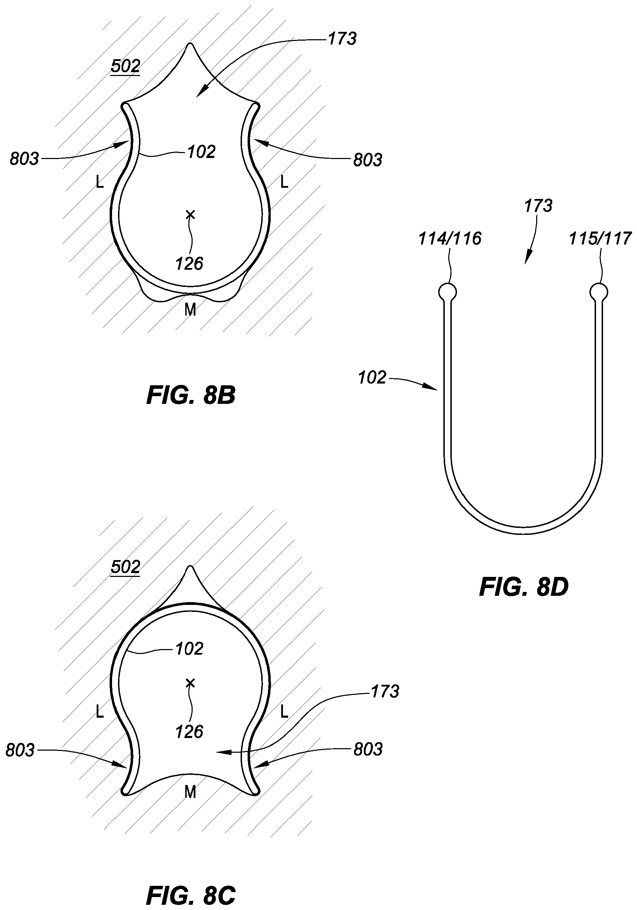

FIG. 8A is a perspective view of another example embodiment according to the invention of implant 102, and FIGs. 8B and 8C are end-on views of this embodiment placed in different orientations within the urethra. This embodiment is open-sided and the portions 803 of implant 102 adjacent the open side, namely, spines 112a and 112b, distal end 801 and proximal end 802, flare radially outwards away from center axis 126 of ring-shaped sections 111. Such a configuration can be referred to as U-shaped with flared ends, omega (Q)-shaped, pineapple-shaped, or otherwise. Embodiments with a flared open side can increase retraction of lateral lobes L as depicted in FIG. 8B, reduce propensity of the implant to rotate, and increase implant fixation force. In the orientation of FIG. 8C, the flared portions 803 of faced posteriorly to embed into the space between the lateral lobes L and median lobe M to improve fixation and prevent urine exposure in the posterior urethra on the sides of the urethral crest.

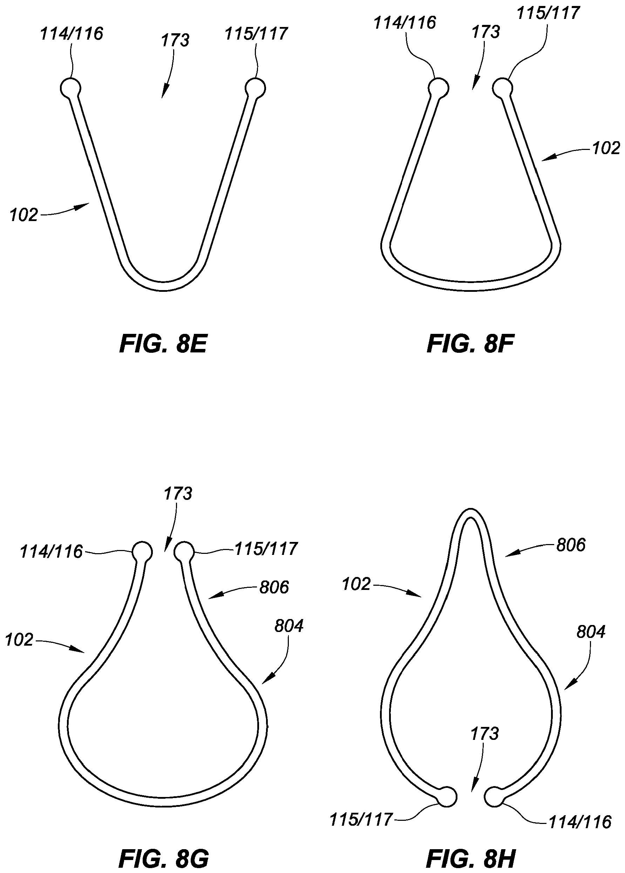

FIGs. 8D-8F are end-on views depicting additional example embodiments of implant 102. Here, implant 102 has a U-shape with open side 173 and enlarged ends 116 and 117. The elongate sides of the U can be straight or curved. In FIG. 8D, the elongate sides of the U-shape are generally parallel, while in FIG. 8E the elongate sides generally diverge from the closed end of the U to open side 173, and in FIG. 8F the elongate sides generally converge from the closed end of the U to open side 173. These embodiments can be positioned in any desired radial orientation (e.g., with enlarged ends 116 and 117 on either side of the median lobe, against a lateral lobe, or against the anterior surface of the prostate).

FIGs. 8G and 8H are end-on views depicting additional example embodiments of implant 102. Here, implant 102 has a general tear-drop shape with a relatively large diameter circular or bulbous section 802 that converges to a relatively narrower tapered section 803. Open side 173 can be positioned at the end of tapered section 803 (FIG. 8G) or at the opposite end in circular section 802. These embodiments can be positioned in any desired radial orientation within the urethra, e.g., with circular section 102 positioned posteriorly against the median lobe and tapered section 803 positioned anteriorly, or vice versa).

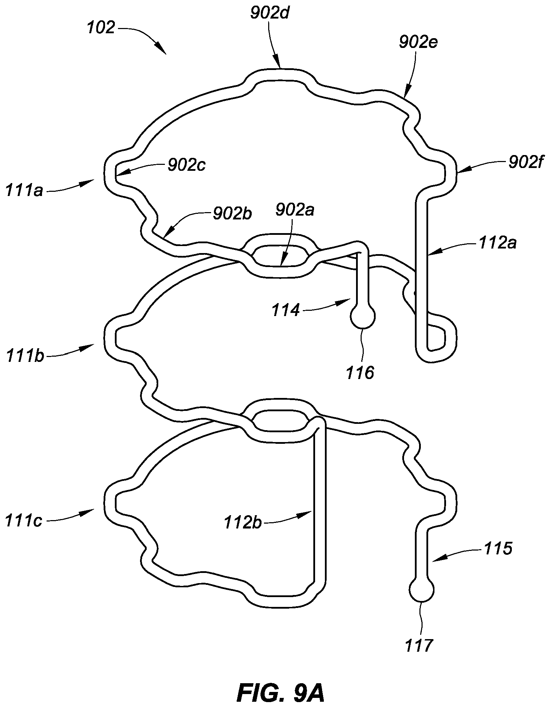

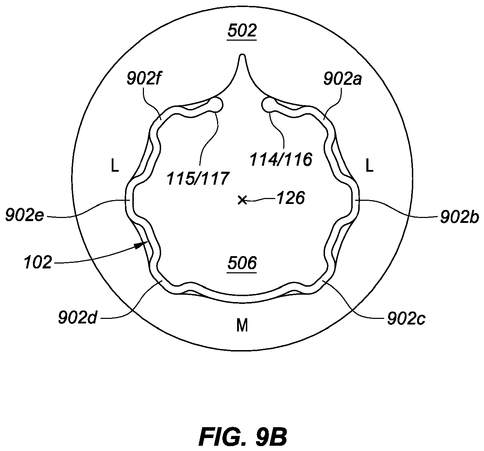

FIG. 9A is a perspective view of another example embodiment of implant 102, and FIG. 9B is an end on view of the embodiment within the urethra. In this embodiment, the main body wire of implant 102 has additional curves or ridges 902 in ring-shaped sections 111. As can be seen from FIG. 9A, each ring-shaped structure 111 has six ridges 902 (labeled as 902a through 902f on structure 111a for convenience). Any number of one or more ridges 902 can be implemented on each ring-shaped structure 111 and/or spine 112. Further, each of structures 111 can have the same, less (including none), or more ridges 902 that the other structures 111 of a particular implant 102, and the same applies for spines 112. These ridges 902 extend radially outwards from what is otherwise a generally constant radius about center axis 126, although in other embodiments, ridges 902 can extend radially inwards or both outwards and inwards.

As can be seen in FIG. 9B, ridges 902 increase the surface area of implant 102 in contact with the surrounding tissue, thus increasing friction between implant 102 and the tissue and improving the ability of implant 102 to remain fixed and not radially rotate within the urethra. Here, ridges 902 are positioned generally in the regions of implant 102 to be placed adjacent to lateral lobes L, while the regions adjacent median lobe M and the anterior prostatic urethra do not have ridges 902.

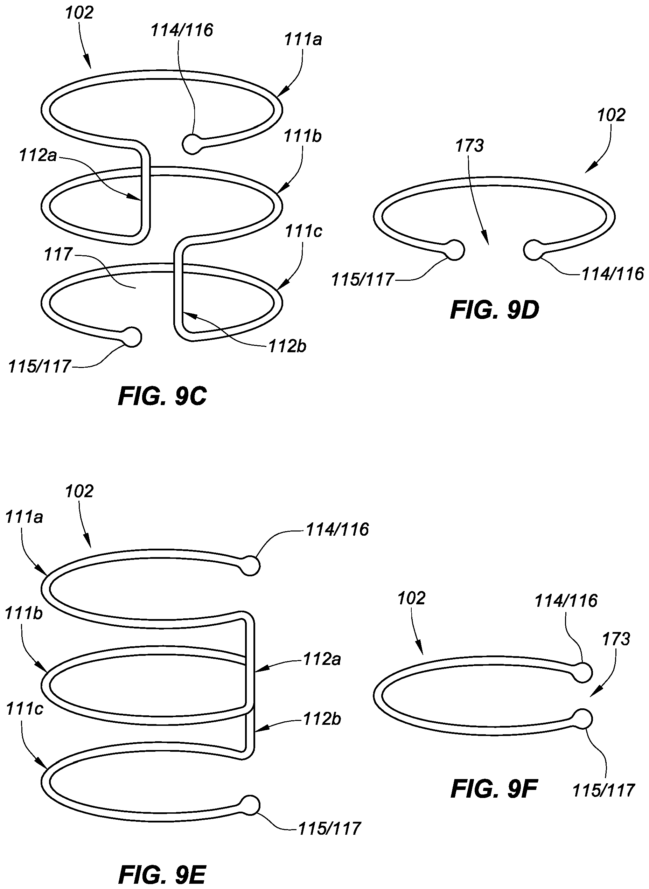

FIGs. 9C and 9D are perspective and end-on views, respectively, depicting a first example embodiment of implant 102 with elliptical ring-shaped structures 111. Here, the elliptical ring-shaped structures 111 have an open side 173 at a portion (bottom) with a relatively low radius of curvature. FIGs. 9E and 9F are perspective and end-on views, respectively, depicting a second example embodiment of implant 102 with elliptical ring-shaped structures 111, but with open side 173 at a portion (right) with a relatively high radius of curvature. These embodiments can be positioned in any desired radial orientation (e.g., with the relatively higher radius portions positioned anteriorly and posteriorly, or with the relatively higher radius portions positioned laterally against lateral lobes).

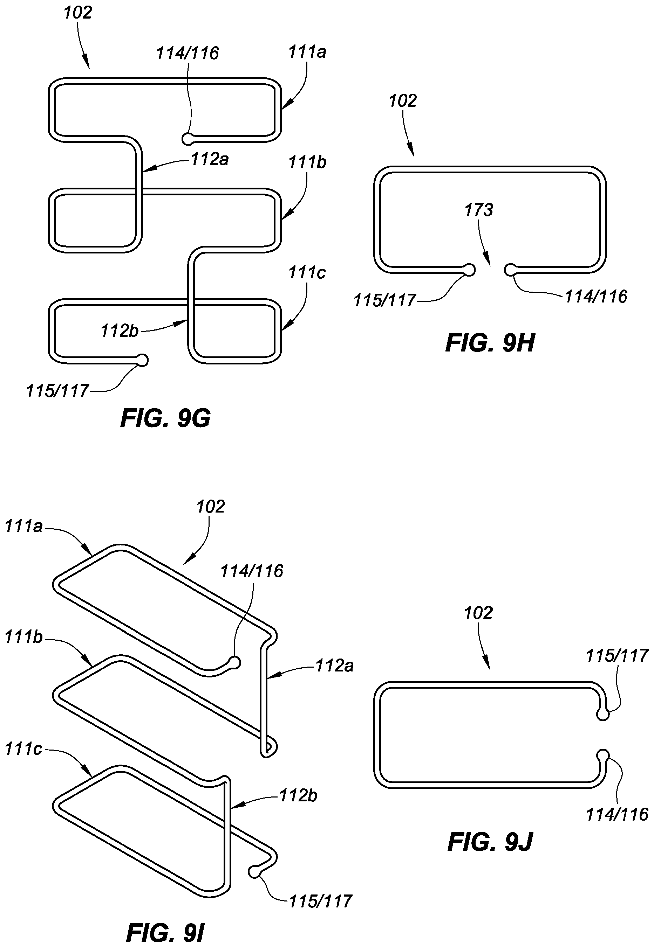

FIGs. 9G and 9H are perspective and end-on views, respectively, depicting a first example embodiment of implant 102 with rectangular ring-shaped structures 111. Here, the rectangular ring-shaped structures 111 have rounded corners with an open side 173 at the center of a relatively longer (bottom) side. FIGs. 9I and 9J are perspective and end-on views, respectively, depicting a second example embodiment of implant 102 with rectangular ring-shaped structures 111, but with open side 173 at the center of a relatively shorter (right) side. Different degrees of rounded corners for the rectangle can be present, including sharp corners with little to no rounding. These embodiments can be positioned in any desired radial orientation (e.g., with the relatively longer sides positioned anteriorly and posteriorly, or with the relatively longer sides positioned laterally against the lateral lobes such that the relatively shorter sides are positioned anteriorly and posteriorly).

In the embodiments described with respect to FIGs. 7A-9J, each ring-shaped structure 111 has a particular shape. These embodiments can be combined such that an implant 102 includes multiple ring-shaped structures 111, each having a different shape (e.g., a non-circular shape combined with a circular shape, and so forth).

In other embodiments, implant 102 can be generally square (where all four sides are equal) or can have other variations of side length that are not square or rectangular (e.g., trapezoidal). Implant 102 can also be configured with more than four sides and can have five sides (e.g., pentagonal), six sides (e.g., hexagonal), seven sides (e.g., heptagonal), eight sides (e.g., octagonal), nine sides (e.g., nonagonal), and so forth.

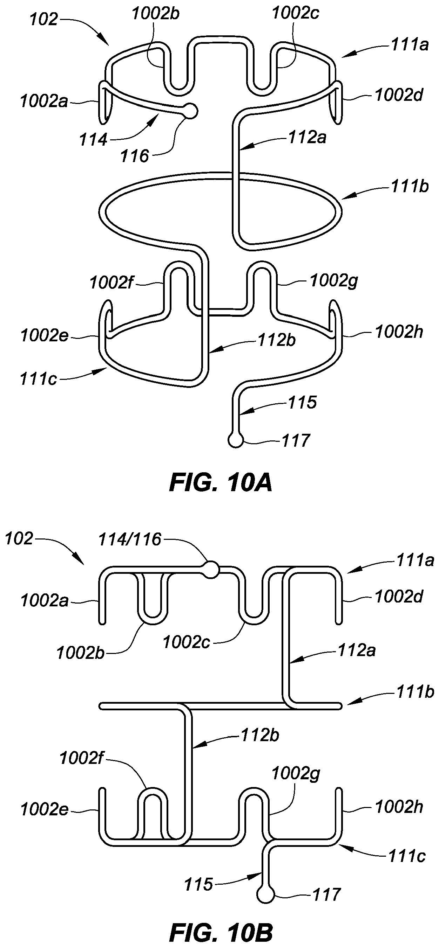

FIGs. 10A-10E depict additional example embodiments of implant 102 configured to maintain radial orientation within the urethra. In these embodiments, longitudinal axis projections or tabs are present to assist in maintaining implant 102 in a fixed radial orientation and, in some embodiments, also for increased retraction of the lateral lobes. Such features may allow for increased ring spacing without sacrificing the ability of the implant to provide durable obstruction relief. Implant 102 can be configured with one or more such longitudinal projections or tabs. Each ring-shaped structure 111 can have none, one, or more tabs depending on the desired configuration. Each tab can extend distally and/or proximally. Tabs can extend from ring-shaped structures for the same distances or different distances on an implant. Tabs can be formed directly from the main wire body of implant 102, or can be discrete components connected to the main body by, e.g., welding or adhesive. The tabs can have a U-shape, an S shape, a sinusoidal shape, a flat planar shape, or other shapes. The tabs can be placed on the lateral sides of the implant such that they are in contact with a lateral lobe, or can be placed in more anterior or posterior (median lobe) positions. These tabs can also have teeth, barbs, or other features that puncture into the wall to ensure migration prevention (as described below). Any combination of the aforementioned variations can be implemented.

FIGs. 10A and 10B are perspective and side views, respectively, of an example embodiment of implant 102 with two ring-shaped structures 111a and 111c having longitudinal axis projections or tabs 1002 to assist in maintaining implant 102 in a fixed radial orientation and also for increased retraction of the lateral lobes. Tabs 1002 extend from ring-shaped sections 111 longitudinally along center axis 126 (parallel to urine flow) of the device, as opposed to radial projections that would extend towards or away from center axis 126 (transverse to the direction of urine flow).

More specifically, implant 102 includes four tabs (1002a through 1002d) that extend proximally from distal-most ring-shaped structure 111a and an additional four tabs (1002e through 1002h) that extend distally from proximal-most ring-shaped structure 111c. Intermediate ring-shaped structure 111b has no tabs in this embodiment. Each tab 1002 in this embodiment has a general U-shape such that the main wire body of implant 102 first bends away the ring-shaped structure and then reverses back towards the ring-shaped structure. Ring-shaped structures 111a and 111c each have two tabs (1002b, 1002c, 1002f, 1002g) that are located opposite to the open-side of implant 102, and two tabs (1002a, 1002d, 1002e, 1002h) that are located laterally in position to abut and retract lateral lobes L if the open-side of implant 102 is placed directly anteriorly or proximally.

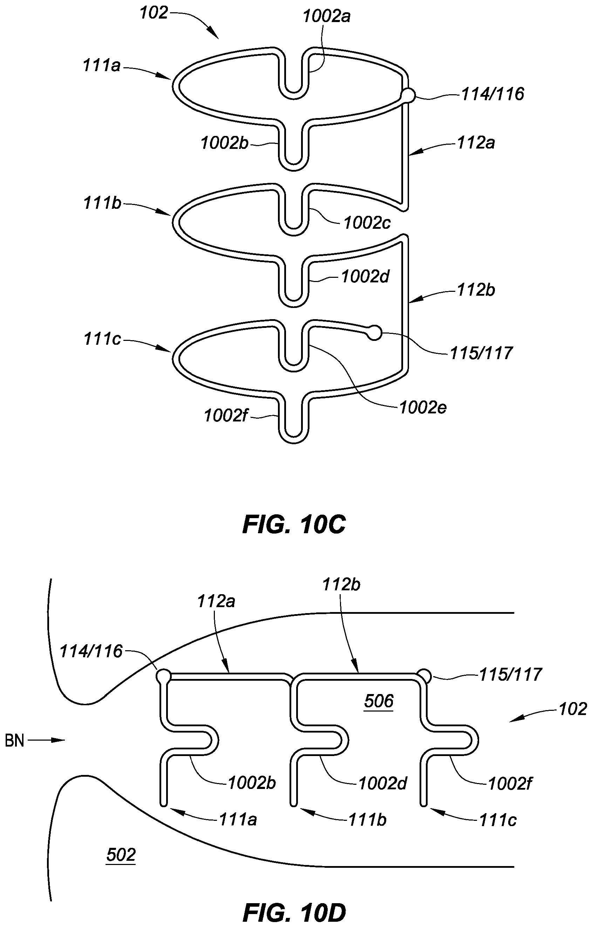

FIG. 10C is a perspective view depicting another example embodiment of implant 102 having tabs 1002. FIG. 10D is a side view of this implant 102 in a sagittal cross-section of the prostatic urethra. Here, each ring-shaped structure 111a, 111b, and 111c has two proximally extending tabs (1002a through 1002f) located on opposite sides in position to abut and retract lateral lobes (located in front and in back of FIG. 10D).

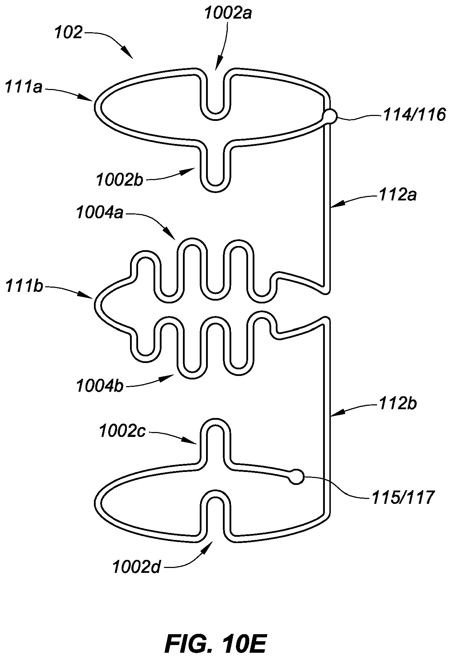

FIG. 10E is a perspective view depicting another example embodiment of implant 102 having tabs 1002. Here, distal-most ring-shaped structure 111a has two proximally extending tabs (1002a, 1002b) located on opposite sides and proximal-most ring-shaped structure 111c has two distally extending tabs (1002c, 1002d) located on opposite sides in position to abut and retract lateral lobes. Intermediate ring-shaped structure 111b has two tab groupings 1004a and 1004b where each tab grouping has a sinusoidal shape extending both distally and proximally from ring-shaped structure 111b. The sinusoidal projections can have the same height or can be at varying heights as shown here.

As described with respect to FIGs. 5E-5H, implant 102 may be placed purposefully to occupy either a more posterior position or a more anterior position within the prostatic urethra. In some embodiments, a barb or other tissue anchor can be included to cause implant 102 to securely capture the desired (posterior or anterior) tissue wall. Example embodiments of barbs and tissue anchors are described below with respect to FIGs. 14A-C.

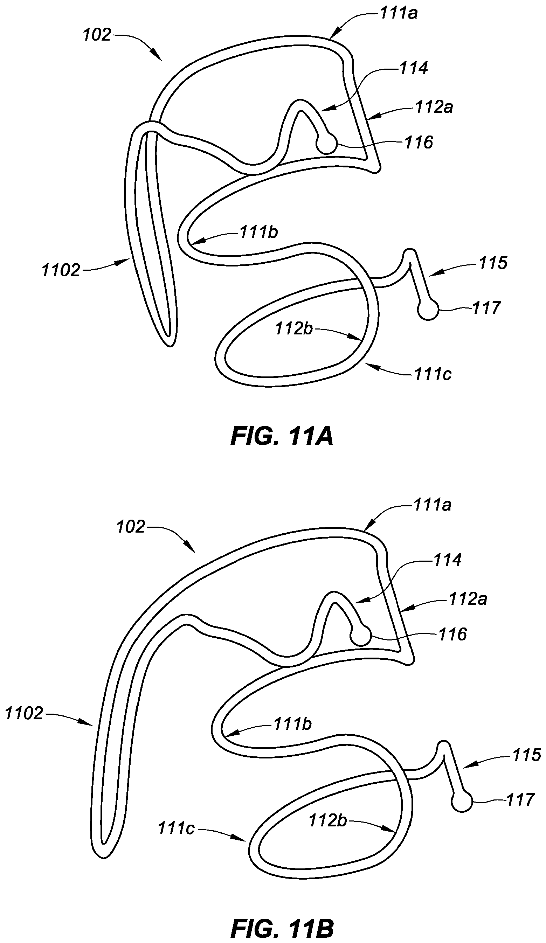

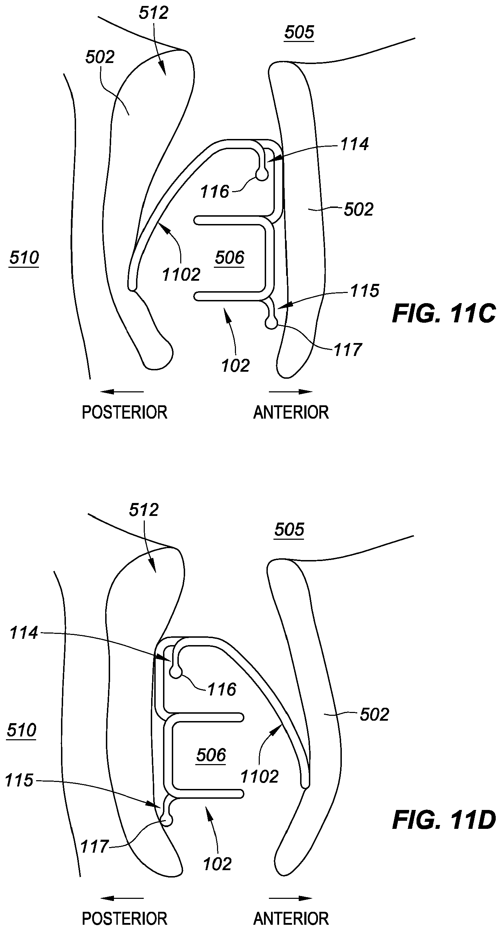

Other positioning mechanisms can be included on implant 102. FIGs. 11A-B depict an example embodiment of an implant 102 having a spring 1102 that can push against the tissue wall and maintain implant 102 in position (anterior or posterior). Here, spring 1102 is in the form of an extension to distal-most ring-shaped structure 111a that curves longitudinally and proximally along the side of implant 102. Spring 1102 can be biased to transition from a compressed state (FIG. 11A) towards an at-rest state (FIG. 11B) to apply force against the tissue wall. FIGs. 11C and 11D are side views of this implant 102 within sagittal cross-sections of the prostatic urethra 502. In FIG. 11C, spring 1102 is exerting force against the posterior wall of prostate 502 to maintain implant 102 in a relatively more anterior position. In FIG. 11D, spring 1102 exerting force against the anterior wall of prostate 502 to maintain implant 102 in a relatively more posterior position. Implant 102 can also be delivered such that spring 1102 pushes against a lateral lobe to force implant 102 against the opposite lateral lobe.

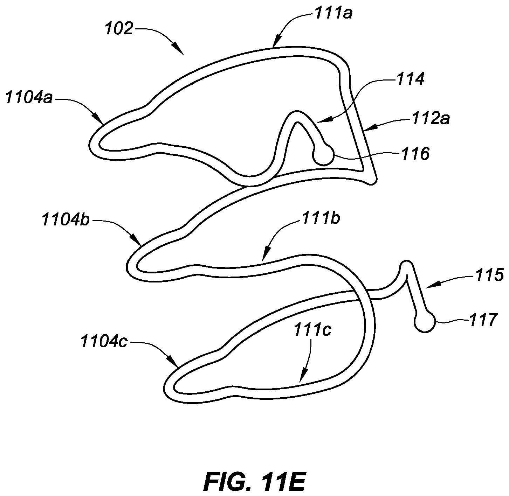

In other embodiments, implant 102 can include one or more projections that project radially away from ring-shaped structures 111 and push against one wall, e.g., like a stilt, to maintain implant 102 against the opposite wall. FIG. 11E is a perspective view depicting another example embodiment of implant 102. Here, each ring-shaped structure 111 includes a projection 1104 that, depending on the implanted orientation, can push against any of the urethra surfaces (anterior wall, posterior wall, lateral lobe) to push implant 102 into a position against the opposite surface. In this embodiment, projection 1104 is a U-shaped extension of ring-shaped structure 111 having a relatively smaller radius of curvature. Other configurations can be used as well, such as a single straight projection from each structure 111.

Migration-prevention may also be achieved with a portion of implant 102 that extends into the bladder neck, and optionally into the bladder. FIG. 12A is a side perspective view depicting an example embodiment of implant 102 with an anchor extension 1202 in the form of a helical coil or spiral, within a sagittal cross-section of urethra 502. An elongate section 1203 extends from distal-most ring-shaped section 111a to helical coil section 1204 having a progressively increasing diameter. Helical coil section 1204 is shown has having four loops but any number of one or more loops can be used. Each more distally located loop can have progressively increasing diameters as shown here, constant diameters, or a combination thereof. The distal-most loop of helical coil section is generally at the interface between the bladder neck BN and the bladder 505. Also shown here are elements of delivery device 103, including balloon anchor 152 in a state of deployment within bladder 505, and anchor member 150, which in turn extends from inner shaft 130. Anchor extension 1202 can be lineated or crimped to fit within delivery device 103.

Other shapes of anchor extension 1202 can also be used, such as a hook-like shape or single loop that reaches in any direction (anterior, posterior, lateral) to prevent migration of tissue and/or implant 102. For patients with an intravesical median lobe, the implant may include an additional ring, loop, or other feature designed to reside within the bladder in contact with the intravesical surface of the median lobe that faces the bladder neck. FIG. 12B is a perspective view depicting an example embodiment of implant 102 with anchor extension 1202 in the form of a hook or strut. Anchor extension 1202, in this embodiment, is an elongate U-shape formed from distal-most ring-shaped structure 111a, with a direction that extends distally and laterally from structure 111a. Extension 1202 can be lineated to the diameter of a single wire along with the rest of implant 102 to fit within delivery device 103.

FIGs. 12C and 12D are side views of implant 102 within a sagittal cross-section of prostatic urethra 502 and bladder 505. Anchor extension 1202, in this embodiment, is adapted to extend through bladder neck BN and over tissue of or near the bladder wall, which in this example is intravesical median lobe 514, which is sometimes referred to as a ball-valve. Anchor extension 1202 can serve to maintain intravesical median lobe 514 in a position away from bladder neck BN, and thus prevent obstruction of bladder neck BN. Anchor extension 1202 can also serve as an anchor to maintain positioning of implant 102 within prostatic urethra 502, and implant 102 can also maintain extension 1202 in the proper position. The medical professional can select the appropriate length of anchor extension 1202 to accommodate different tissue profiles. FIG. 12C depicts an example where anchor extension 1202 grasps or abuts a portion (e.g., about half) of the surface of lobe 514 facing bladder neck BN, and FIG. 12D depicts an example where anchor extension 1202 grasps or abuts the entire surface of lobe 514 facing bladder neck BN. Other length variations can be implemented, as well as shape variations like, for example, where extension 1202 is in the form of a loop that extends around lobe 514.

In some embodiments, implant 102 can be designed to relieve obstruction in patients with lateral lobe hyperplasia and/or median lobe hyperplasia within the prostatic urethra. For these indications, implant 102 preferably resides entirely within the prostatic urethra.

FIGs. 13A and 13B are perspective and side views, respectively, of another example embodiment of implant 102. Here, implant 102 has a laterally-symmetric multiple-ring configuration with ring-shaped structures 111 interconnected by spines 112 in alternating side placements. Specifically, distal-most ring-shaped structure 111a is coupled with spines 112a and 112b located adjacent each other on a first (top) side of implant 102. The next proximal ring-shaped structure 111b (composed of two portions not directly connected) is coupled with spines 112a and 112b on the first side and spines 112c and 112d on the opposite second side (bottom) of implant 102. The next proximal ring-shaped structure 111c (again composed of two portions not directly connected) is coupled with spines 112c and 112d on the second side and spines 112e and 112f on the first side (top) of implant 102. Proximal-most ring-shaped structure 111d is coupled with spines 112e and 112f. The lateral symmetry of this embodiment is best seen in FIG. 13B, which also shows that ring-shaped structures 111 are slightly non-planar (e.g., curvilinear) and angled with respect to each other, although other configurations can be implemented where ring-shaped structures 111 are parallel and/or planar.

In this embodiment (and the others shown herein without engagement features), enlarged bodies 116 and 117 can be positioned on implant 102 at desired locations for mating with the delivery device. Here, enlarged bodies 116 and 117 are coupled with ring-shaped structures 111a and 111d at the second (bottom) side. These bodies 116 and 117 can be placed in other positions on implant 102 as desired.

In all the embodiments of implant 102 described herein, the implant body may have one or more features that increase friction with the surrounding tissue. These features can range from an abrasive or textured surface to pronounced projections or anchors such as barbs, cleats, spikes, globular or ball-like projections, ridges, grooves, and the like. These features can provide small areas of point contact which act to anchor the implant to the urethral wall at a particular point.

Implant 102 can be configured to have one or more anchors on one or more of ring-shaped structures 111, tabs 1002, and/or spines 112. FIG. 14A is a perspective view depicting an example embodiment of implant 102 with radially oriented anchors 1402 to resist rotation or axial migration. Each anchor 1042 can have a tapered or pointed end to pierce the adjacent tissue. In this embodiment, ring shaped structure 111 includes three anchors 1402a, 1402b, and 1402c. Anchors 1402 can be formed from discrete bodies that are then attached to the main body of implant 102, or anchors 1402 can be formed directly from the implant body. FIGs. 14B and 14C depict an example embodiment where an anchor 1402 is formed from the implant body. In FIG. 14B, a section of the implant body 1404 (e.g., a cylindrical section of wire) has a partial cut, slit, or gap 1405 at an acute angle 1406 (e.g., between about 15 and 60 degrees) from the long axis 1407 of the section. When the wire is formed into a circular or rounded shape or deployed from lineated to the circular or rounded shape, the anchor portion 1402 separates from body 1404 and transitions into a position where it can lodge in the tissue (beyond the normal diameter of structure 111).

In all of the embodiments of implant 102 described herein, the wire element forming the main body of implant 102 can have any of a host of cross-sectional shapes. A common example of the main body wire element is cylindrical wire with a circular cross-section. Other wire shapes can be used, e.g., to provide more stability against the lobular forces of the prostatic urethra. Such other shapes include, but are not limited to: wire with an elliptical or other rounded but non-circular cross-section; wire with a polygonal (three or more sides) cross-section having rounded or sharp corners that is, e.g., triangular, square, rectangular (e.g., ribbon wire), trapezoidal, pentagonal, hexagonal, and the like; or a combination of the rounded and multi-sided wire shapes (e.g., a D-shaped cross-section). If ribbon wire with a rectangular cross-section is used, the wider side of the ribbon can face the urethral wall (see FIG. 15). When used for ring-shaped sections 111, ribbon wire can ensure the ring-shaped structure 111 remains in-plane and concentric with the prostatic urethral lumen. This non-circular cross section may exist along the entire implant, or selectively at any section along the implant where stability is required. FIG. 15 is a perspective view of an example embodiment of implant 102 where ring-shaped sections 111a, 111b, and 111c are formed from ribbon wire having a rectangular cross-section and spines 112a and 112b are formed from cylindrical wire having a circular cross-section. The body of this implant 102 can be shaped from one wire to give both types of cross-sections, can be formed by welding cylindrical wire to ribbon wire, or otherwise.

In all of the embodiments described herein, each expander section 111 of implant 102 can have a width or diameter (the same or different) that is between 8 and 24 mm. In embodiments with more uniformity, each expander section 111 can have a width or diameter (the same or different) that is between 12 and 18 mm. Still other ring diameters are within the scope of this disclosure. In all of the embodiments described herein, the main body (e.g., the single wire) of implant 102 can have a maximum width or diameter (constant or varying) between 0.40 and 0.90 mm, while certain more uniform embodiments can have a width or diameter between 0.50 and 0.75 mm. Still other wire diameters are within the scope of this disclosure.

The spacing between expander sections 111 can be varied such that implant 102 effectively holds back the encroaching prostate while maintaining the implant's ability to embed and invaginate into the urethral wall to prevent urine exposure. To achieve both these goals, many embodiments can have a spacing between expander structures 111 of between 4 and 14 mm. If more uniformity is desired, implant 102 can have a spacing between 6 and 10 mm. Still other spacings are within the scope of this disclosure. In some embodiments, a single implant 102 may have different spacings between different structures 111, whereas in other embodiments, the spacing may be uniform.

In all of the embodiments of implant 102 described herein, the main body of implant 102 can be a single wire that is capable of being lineated into a straight wire having no more than slight kinks, oscillations, or bends (e.g., see FIG. 3A) resulting from the instilled bias, and without any loop or other shape where the single wire reverses back upon itself (e.g., in a U shape, O shape, etc.). Stated another way, the single wire is not securely coupled to itself (e.g., with a weld or fastener) and is not securely coupled to another structure (e.g., a hub, collar, scaffold, sheet, tissue graft, and the like) incapable of being lineated. (Exceptions may be made for structures that have a width similar to the diameter of the single wire itself, as such has little impact on the minimum dimension implant 102 can be reduced to for fitting within the delivery device.) While implant 102 can be biased towards the laterally expanded at-rest configuration, such embodiments remain lineatable back to a straight or substantially straight configuration.

In all of the embodiments described herein, implant 102 can include no engagement features, only a distal engagement feature 114 (with or without enlarged body 116), only a proximal engagement feature 115 (with or without enlarged body 117), or both. Engagement features 114 and 115 can have the same width or diameter as the main body or a smaller width or diameter (see, e.g., FIGs. 6A, 6B, and 6F). Each engagement feature 114 and 115 can extend proximally (see, e.g., FIGs. 1B-1D), or distally, or a combination of the two (see, e.g., FIG. 6A). Each engagement feature 114 and 115 can also extend radially (see, e.g., FIGs. 6B and 6F). Enlarged ends 116 and 117 of the engagement features 114 and 115 can be formed or attached directly on a ring-shaped structure 111 or a spine 112, without an intervening segment.

In all of the embodiments described herein, the implant can be implanted within the urethra such that an open side of the implant is positioned against a posterior surface of the urethra (e.g., a median lobe), an anterior surface of the urethra (e.g., opposite the median lobe), or a lateral surface of the urethra (e.g., the left or right lateral lobe). When positioned against the posterior surface of the urethra, the ends of the open side (when viewed end-on in a cross-section perpendicular to the direction of urine flow) can be positioned on opposite sides of the medial lobe. In all of the embodiments described herein, the implant can be implanted such that it rests against only a posterior surface and one or both lateral surfaces of the urethra (and not against an anterior surface). In all of the embodiments described herein, the implant can be implanted such that it rests against only an anterior surface and one or both lateral surfaces of the urethra (and not against an anterior surface). Other placements are possible as well.

Although already explained, for the sake of clarity the implant embodiments described herein may vary in features and size parameters to treat various subcategories of anatomical configurations such as varying prostate volume, prostatic urethral length, prostatic urethral anterior-posterior height, degree of prostatic urethral curvature, bladder neck geometry, desire of patient to maintain sexual function including antegrade ejaculation, and other factors.

In all of the embodiments described herein, implant 102 can be formed from a host of different materials. In some embodiments, implant 102 has a passive bare-metal construction. In some embodiments, implant 102 is composed of nitinol, such as electropolished and passivated nitinol, or a different processing or surface finish. In other embodiments, implant 102 is formed of stainless steel, cobalt chromium, or a polymer. Other materials may be used. Surface finishes, platings, or coatings can be applied, such as gold or silver plating, polymer coating, hydrophilic or hydrophobic coating (e.g., polyvinylpyrrolidone (PVP), polytetrafluoroethylene (PTFE), silicone, etc.), and others. Implant 102 can include mechanisms or coatings for drug elution, coatings to prevent encrustation, coatings to minimize adverse tissue response, or to promote or prevent epithelialization of the implant.

Implant 102 can be configured as an inductive coil such that implant 102 can act as a receiver for energy transmitted from outside of the patient. The received energy may be applied to heat or cool the surrounding tissue, to power electronic components within or adjoining the implant, or for other uses.

The embodiments described herein are restated and expanded upon in the following paragraphs without explicit reference to the figures. In many example embodiments, an implant for placement in a prostatic urethra lumen is provided, the implant including: an elastic wire body adapted to laterally expand and longitudinally contract from a deployment configuration to an at-rest configuration, wherein the elastic wire body is biased towards the at-rest configuration, the at-rest configuration configured to maintain the prostatic urethra lumen in an at least partially open state, wherein the elastic wire body in the at-rest configuration has a plurality of ring-shaped structures that are non-coplanar, the ring-shaped structures located about a longitudinal axis of the implant, and wherein each of the plurality of ring-shaped structures is coupled with another of the plurality of ring-shaped structures by an interconnect portion of the elastic wire body, the interconnect portion extending longitudinally such that the plurality of ring-shaped structures are spaced apart.

The implant can include a distal engagement member and/or a proximal engagement member. In some embodiments, in the at-rest configuration, the distal engagement member can extend proximally from a distal-most ring-shaped structure, and the proximal engagement member can extend distally from a proximal-most ring-shaped structure. In some embodiments, in the at-rest configuration, the distal engagement member can extend proximally and radially from a distal-most ring-shaped structure, and the proximal engagement member can extend distally and radially from a proximal-most ring-shaped structure. In some embodiments, the distal engagement member has an enlarged terminus and/or the proximal engagement member has an enlarged terminus. In some embodiments, each enlarged terminus is ball-shaped. In some embodiments, in the at-rest configuration, the distal engagement member comprises a terminus and the distal engagement member extends radially inwards such that the terminus is in a central interior space of the implant. In some embodiments, the terminus is at a radial center of the implant. In some embodiments, the elastic wire body has a main diameter, and the distal engagement member and/or proximal engagement member taper to smaller diameters than the main diameter.

In some embodiments, in the at-rest configuration, the elastic wire body extends 360 degrees around the longitudinal axis. In other embodiments, in the at-rest configuration, the elastic wire body extends less than 360 degrees around the longitudinal axis such that the implant has an open side. The elastic wire body can extend less than 360 degrees and 270 degrees or more around the longitudinal axis such that the implant has an open side.

In the at-rest configuration, at least one ring-shaped structure can have a triangular radial shape with three sides. In some embodiments, the triangular radial shape can have a radial opening along one of the three sides of the triangular shape. In some embodiments, the triangular radial shape has a radial opening at a corner of the triangular shape.

In the at-rest configuration, at least one ring-shaped structure can have a U radial shape with elongate sides, a closed end and an open end. In some embodiments, the elongate sides are parallel. In some embodiments, the elongate sides converge towards the open end. In some embodiments, the elongate sides diverge towards the open end. In some embodiments, ends of the elongate sides flare radially outwards.

In the at-rest configuration, at least one ring-shaped structure can have a tear drop radial shape comprising a tapered section and a relatively wider diameter section. In some embodiments, a radial open side is present at an end of the tapered section. In some embodiments, a radial open side is present at the relatively wider diameter section opposite the tapered section.

In the at-rest configuration, at least one ring-shaped structure can have a plurality of ridges that project radially outwards.

In the at-rest configuration, at least one ring-shaped structure can have an elliptical or rectangular shape.

In the at-rest configuration, at least one ring-shaped structure can include a longitudinal projection that extends distally and/or proximally. In some embodiments, the longitudinal projection is U-shaped or sinusoidal.

In the at-rest configuration, a distal-most ring-shaped structure can include a longitudinal projection that extends proximally, and a proximal-most ring-shaped structure comprises a longitudinal projection that extends distally. In some embodiments, an intermediate ring-shaped structure between the distal-most and proximal-most ring-shaped structures does not include a longitudinal projection.

The implant can include a spring biased towards a laterally projecting state. In some embodiments, a distal-most ring-shaped structure comprises an extension that is the spring.

The implant can include an anchor extension that extends distally from a distal-most ring-shaped structure. In some embodiments, the anchor extension comprises a helical coil. In some embodiments, the helical coil includes a plurality of loops, the distal-most loop having a greater diameter than the proximal-most loop.

In the at-rest configuration, the elastic wire body of the implant can be laterally symmetrical. In some embodiments, a first end of a distal-most ring-shaped structure is connected to a first interconnect portion, a second end of the distal-most ring-shaped structure is connected to a second interconnect portion, a first end of a proximal-most ring-shaped structure is connected to a third interconnect portion, and a second end of the proximal-most ring-shaped structure is connected to a fourth interconnect portion. In some embodiments, an intermediate ring-shaped structure between the distal-most and proximal-most ring-shaped structures is formed of two unconnected portions.

The implant can include at least one ring-shaped structure with a tissue anchor. In some embodiments, the anchor is configured as a radially projecting barb.

The elastic wire body can have a first portion with a circular wire cross-section and a second portion with a non-circular wire cross-section. In some embodiments, the non-circular wire cross-section has a shape selected from: rectangular, D-shaped, trapezoidal, and elliptical.

Many embodiments of a method of implanting an implant in a prostatic urethra lumen are provided, and the method can include: advancing a delivery device into a urinary tract of a patient; and deploying the implant from the delivery device such that the implant laterally expands and longitudinally contracts from a deployment configuration towards an at-rest configuration and at least partially resides in the prostatic urethra, wherein the implant comprises an elastic wire body biased towards the at-rest configuration, the at-rest configuration configured to maintain the prostatic urethra in an at least partially open state, wherein the elastic wire body in the at-rest configuration has a plurality of ring-shaped structures that are non-coplanar, the ring-shaped structures located about a longitudinal axis of the implant, and wherein each of the plurality of ring-shaped structures is coupled with another of the plurality of ring-shaped structures by an interconnect portion of the elastic wire body, the interconnect portion extending longitudinally such that the plurality of ring-shaped structures are spaced apart. The method can be performed with any and all of the implant embodiments described herein.

The method can include releasing the implant from the delivery device by disengaging a distal engagement member and a proximal engagement member of the implant. In some embodiments, in the at-rest configuration, the distal engagement member extends proximally from a distal-most ring-shaped structure, and the proximal engagement member extends distally from a proximal-most ring-shaped structure. In some embodiments, in the at-rest configuration, the distal engagement member extends proximally and radially from a distal-most ring-shaped structure, and the proximal engagement member extends distally and radially from a proximal-most ring-shaped structure. In some embodiments, in the at-rest configuration, the distal engagement member comprises a terminus and the distal engagement member extends radially inwards such that the terminus is in a central interior space of the implant. In some embodiments, the terminus is at a radial center of the implant. In some embodiments, the elastic wire body has a main diameter, and wherein the distal engagement member and proximal engagement member taper to smaller diameters than the main diameter.

The implant can be deployed from the delivery device such that the open side is positioned on an anterior side, a posterior side, or a lateral side of the prostatic urethra.

In the at-rest configuration, at least one ring-shaped structure can have a triangular radial shape with three sides. In some embodiments, the implant can be deployed from the delivery device such that a corner of the triangular radial shape is positioned on an anterior side of the prostatic urethra and the side opposite the corner is positioned on a posterior side of the prostatic urethra. In some embodiments, the implant can be deployed from the delivery device such that a corner of the triangular radial shape is positioned on a posterior side of the prostatic urethra and the side opposite the corner is positioned on an anterior side of the prostatic urethra.

In the at-rest configuration, at least one ring-shaped structure can have a U radial shape with elongate sides, a closed end and an open end. In some embodiments, the implant is deployed from the delivery device such that the closed end is positioned on an anterior side of the prostatic urethra and the open end is positioned on a posterior side of the prostatic urethra. In some embodiments, the implant is deployed from the delivery device such that the closed end is positioned on a posterior side of the prostatic urethra and the open end is positioned on an anterior side of the prostatic urethra. In some embodiments, ends of the elongate sides flare radially outwards and the implant is deployed from the delivery device such that each end of the elongate sides is positioned between a lateral lobe and a medial lobe of the prostatic urethra.

In the at-rest configuration, at least one ring-shaped structure can have a tear drop radial shape comprising a tapered section and a relatively wider diameter section. In some embodiments, the implant is deployed from the delivery device such that the tapered section is positioned on a posterior side of the prostatic urethra and the relatively wider diameter section is positioned on an anterior side of the prostatic urethra. In some embodiments, the implant is deployed from the delivery device such that the tapered section is positioned on an anterior side of the prostatic urethra and the relatively wider diameter section is positioned on a posterior side of the prostatic urethra.

In the at-rest configuration, at least one ring-shaped structure has a plurality of ridges that project radially outwards. In some embodiments, the implant is deployed from the delivery device such that the plurality of ridges are positioned on a lateral side of the prostatic urethra. In some embodiments, the implant is deployed from the delivery device such that the plurality of ridges are positioned on a posterior side of the prostatic urethra. In some embodiments, the implant is deployed from the delivery device such that the plurality of ridges are positioned on an anterior side of the prostatic urethra.

In the at-rest configuration, at least one ring-shaped structure can have an elliptical shape. In some embodiments, the implant is deployed from the delivery device such that opposing sides of the elliptical shape having relatively greater radii of curvature are positioned on lateral sides of the prostatic urethra. In some embodiments, the implant is deployed from the delivery device such that opposing sides of the elliptical shape having relatively greater radii of curvature are positioned on an anterior side and a posterior side of the prostatic urethra.

In the at-rest configuration, at least one ring-shaped structure can have a rectangular shape. In some embodiments, the implant is deployed from the delivery device such that relatively longer sides of the rectangular shape are positioned on lateral sides of the prostatic urethra. In some embodiments, the implant is deployed from the delivery device such that relatively longer sides of the rectangular shape are positioned on an anterior side and a posterior side of the prostatic urethra.

In the at-rest configuration, at least one ring-shaped structure can include a longitudinal projection that extends distally and/or proximally. In some embodiments, the implant is deployed from the delivery device such that the longitudinal projection is positioned on an anterior side of the prostatic urethra. In some embodiments, the implant is deployed from the delivery device such that the longitudinal projection is positioned on a lateral side of the prostatic urethra. In some embodiments, the implant is deployed from the delivery device such that the longitudinal projection is positioned on a posterior side of the prostatic urethra.

The implant can be deployed from the delivery device such that a spring of the implant transitions towards a laterally projecting state. In some embodiments, the spring, in the laterally projecting state, contacts a posterior surface of the urethra. In some embodiments, the spring, in the laterally projecting state, contacts an anterior surface of the urethra. In some embodiments, the spring, in the laterally projecting state, contacts a lateral surface of the urethra.

The implant can further include an anchor extension that extends distally from a distal-most ring-shaped structure. In some embodiments, the implant is deployed from the delivery device such that the anchor extension is positioned at a bladder neck of the urinary tract. In some embodiments, the implant is deployed from the delivery device such that the anchor extension is positioned though a bladder neck and against a surface of an intravesical median lobe in a bladder. In some embodiments, the anchor extension maintains the intravesical median lobe, that can obstruct the bladder neck, in a position that does not obstruct the bladder neck.

As used herein and in the appended claims, the singular forms "a," "an," and "the" include plural referents unless the context clearly dictates otherwise.

## Claims

1. An implant (102) for placement in a prostatic urethra lumen, comprising:
an elastic wire body adapted to laterally expand and longitudinally contract from a deployment configuration to an at-rest configuration, wherein the elastic wire body is biased towards the at-rest configuration, the at-rest configuration configured to maintain the prostatic urethra lumen in an at least partially open state,
wherein the elastic wire body in the at-rest configuration has a plurality of ring-shaped structures (111a, 11b, 11c) that are non-coplanar, the ring-shaped structures located about a longitudinal axis (126) of the implant, and
wherein each of the plurality of ring-shaped structures is coupled with another of the plurality of ring-shaped structures by an interconnect portion (112a, 112b) of the elastic wire body, the interconnect portion extending longitudinally such that the plurality of ring-shaped structures are spaced apart
**characterised in that** at least one of the ring-shaped structures in the at-rest configuration comprises a U-shaped structure with flared ends (803).

2. The implant (102) of claim 1, further comprising a distal engagement member (114) and a proximal engagement member (115).

3. The implant (102) of claim 2, wherein, in the at-rest configuration, the distal engagement member (114) extends proximally from a distal-most ring-shaped structure (111a), and/or the proximal engagement member (115) extends distally from a proximal-most ring-shaped structure (111c).

4. The implant (102) of claim 2, wherein, in the at-rest configuration, the distal engagement member (114) extends proximally and radially from a distal-most ring-shaped structure (111a), and/or the proximal engagement member (115) extends distally and radially from a proximal-most ring-shaped structure (111c).

5. The implant (102) of claims 2-4, wherein the distal engagement member (114) has an enlarged terminus (116) and the proximal engagement member (115) has an enlarged terminus (117).

6. The implant (102) of claim 5, wherein each enlarged terminus (116, 117) is ball-shaped.

7. The implant (102) of claim 2, wherein, in the at-rest configuration, the proximal engagement member (115) comprises a terminus and the proximal engagement member extends radially inwards such that the terminus is in a central interior space of the implant.

8. The implant (102) of claim 1, wherein, in the at-rest configuration, the elastic wire body extends less than 360 degrees around the longitudinal axis such that the implant has an open side.

9. The implant (102) of claim 1, wherein, in the at-rest configuration, the elastic wire body extends less than 360 degrees and 270 degrees or more around the longitudinal axis such that the implant has an open side.

10. The implant of any preceding claim, wherein elongate sides of the at least one ring-shaped structure converge towards the open end.

11. The implant of any preceding claim, wherein ends of elongate sides of the at least one ring-shaped structure flare radially outwards.

12. The implant (102) of any of the foregoing claims, wherein the elastic wire body is nitinol.

## Patentansprüche

1. Implantat (102) zur Platzierung in einem Lumen einer prostatischen Harnröhre, umfassend:
eine elastischen Drahtkörper, der dazu eingerichtet ist, sich aus einer Einsatzkonfiguration in eine Ruhekonfiguration seitlich auszudehnen und längs zusammenzuziehen, wobei der elastische Drahtkörper zu der Ruhekonfiguration hin vorgespannt ist, wobei die Ruhekonfiguration dazu konfiguriert ist, das Lumen der prostatischen Harnröhre in einem zumindest zum Teil offenen Zustand zu halten,
wobei der elastische Drahtkörper in der Ruhekonfiguration eine Vielzahl von ringförmigen Strukturen (111a, 11b, 11c) aufweist, die nicht koplanar sind, wobei die ringförmigen Strukturen um eine Längsachse (126) des Implantats angeordnet sind, und
wobei jede der Vielzahl von ringförmigen Strukturen durch einen Zwischenverbindungsteil (112a, 112b) des elastischen Drahtkörpers mit einer anderen der Vielzahl von ringförmigen Strukturen gekoppelt ist, wobei der Zwischenverbindungsteil sich längs erstreckt, so dass die Vielzahl von ringförmigen Strukturen voneinander beabstandet ist,
**dadurch gekennzeichnet, dass** mindestens eine der ringförmigen Strukturen in der Ruhekonfiguration eine U-förmige Struktur mit aufgeweiteten Enden (803) umfasst.

2. Implantat (102) nach Anspruch 1, weiterhin umfassend ein distales Eingriffselement (114) und ein proximales Eingriffselement (115).

3. Implantat (102) nach Anspruch 2, wobei in der Ruhekonfiguration das distale Eingriffselement (114) sich proximal von der am meisten distalen ringförmigen Struktur (111a) erstreckt und/oder das proximale Eingriffselement (115) sich distal von der am meisten proximalen ringförmigen Struktur (111c) erstreckt.

4. Implantat (102) nach Anspruch 2, wobei in der Ruhekonfiguration das distale Eingriffselement (114) sich proximal und radial von der am meisten distalen ringförmigen Struktur (111a) erstreckt und/oder das proximale Eingriffselement (115) sich distal und radial von der am meisten proximalen ringförmigen Struktur (111c) erstreckt.

5. Implantat (102) nach den Ansprüchen 2-4, wobei das distale Eingriffselement (114) einen vergrößerten Endpunkt (116) aufweist und das proximale Eingriffselement (115) einen vergrößerten Endpunkt (117) aufweist.

6. Implantat (102) nach Anspruch 5, wobei jeder vergrößerte Endpunkt (116, 117) kugelförmig ist.

7. Implantat (102) nach Anspruch 2, wobei in der Ruhekonfiguration das proximale Eingriffselement (115) einen Endpunkt umfasst und das proximale Eingriffselement sich radial nach innen erstreckt, so dass der Endpunkt in einem zentralen Innenraum des Implantats ist.

8. Implantat (102) nach Anspruch 1, wobei in der Ruhekonfiguration der elastische Drahtkörper sich um weniger als 360 Grad um die Längsachse erstreckt, so dass das Implantat eine offene Seite aufweist.

9. Implantat (102) nach Anspruch 1, wobei in der Ruhekonfiguration der elastische Drahtkörper sich um weniger als 360 Grad und 270 Grad oder mehr um die Längsachse erstreckt, so dass das Implantat eine offene Seite aufweist.

10. Implantat nach einem vorhergehenden Anspruch, wobei längliche Seiten der mindestens einen ringförmigen Struktur zu dem offenen Ende hin zusammenlaufen.

11. Implantat nach einem vorhergehenden Anspruch, wobei Enden von länglichen Seiten der mindestens einen ringförmigen Struktur sich radial nach außen aufweiten.

12. Implantat (102) nach einem der vorhergehenden Ansprüche, wobei der elastische Drahtkörper Nitinol ist.

## Revendications

1. Implant (102) destiné à être placé dans une lumière de l'urètre prostatique, comprenant :
un corps en fil métallique élastique conçu pour se dilater latéralement et se contracter longitudinalement en passant d'une configuration de déploiement à une configuration de repos, le corps en fil métallique élastique étant sollicité vers la configuration de repos, la configuration de repos étant configurée pour maintenir la lumière de l'urètre prostatique dans un état au moins partiellement ouvert,
dans lequel le corps en fil métallique élastique dans la configuration de repos comporte une pluralité de structures en forme d'anneau (111a, 11b, 11c) qui ne sont pas coplanaires, les structures en forme d'anneau étant situées autour d'un axe longitudinal (126) de l'implant, et
dans lequel chacune de la pluralité de structures en forme d'anneau est couplée à une autre de la pluralité de structures en forme d'anneau par une partie d'interconnexion (112a, 112b) du corps en fil métallique élastique, la partie d'interconnexion s'étendant longitudinalement de telle sorte que la pluralité de structures en forme d'anneau soit espacée,
**caractérisé en ce qu'**au moins une des structures en forme d'anneau dans la configuration de repos comprend une structure en forme de U ayant des extrémités évasées (803).

2. Implant (102) selon la revendication 1, comprenant en outre un élément d'engagement distal (114) et un élément d'engagement proximal (115).

3. Implant (102) selon la revendication 2, dans lequel, dans la configuration de repos, l'élément d'engagement distal (114) s'étend proximalement à partir d'une structure en forme d'anneau la plus distale (111a), et/ou l'élément d'engagement proximal (115) s'étend distalement à partir d'une structure en forme d'anneau la plus proximale (111c).

4. Implant (102) selon la revendication 2, dans lequel, dans la configuration de repos, l'élément d'engagement distal (114) s'étend proximalement et radialement à partir d'une structure en forme d'anneau la plus distale (111a), et/ou l'élément d'engagement proximal (115) s'étend distalement et radialement à partir d'une structure en forme d'anneau la plus proximale (111c).

5. Implant (102) selon les revendications 2 à 4, dans lequel l'élément d'engagement distal (114) a une extrémité élargie (116) et l'élément d'engagement proximal (115) a une extrémité élargie (117).

6. Implant (102) selon la revendication 5, dans lequel chaque extrémité élargie (116, 117) est en forme de boule.

7. Implant (102) selon la revendication 2, dans lequel, dans la configuration de repos, l'élément d'engagement proximal (115) comprend une extrémité et l'élément d'engagement proximal s'étend radialement vers l'intérieur de telle sorte que l'extrémité se trouve dans un espace intérieur central de l'implant.

8. Implant (102) selon la revendication 1, dans lequel, dans la configuration de repos, le corps en fil métallique élastique s'étend sur moins de 360 degrés autour de l'axe longitudinal de telle sorte que l'implant ait un côté ouvert.

9. Implant (102) selon la revendication 1, dans lequel, dans la configuration de repos, le corps en fil métallique élastique s'étend sur moins de 360 degrés et sur 270 degrés ou plus autour de l'axe longitudinal de telle sorte que l'implant ait un côté ouvert.

10. Implant selon l'une quelconque des revendications précédentes, dans lequel les côtés allongés de l'au moins une structure en forme d'anneau convergent vers l'extrémité ouverte.

11. Implant selon l'une quelconque des revendications précédentes, dans lequel les extrémités des côtés allongés de l'au moins une structure en forme d'anneau s'évasent radialement vers l'extérieur.

12. Implant (102) selon l'une quelconque des revendications précédentes, dans lequel le corps en fil métallique élastique est en nitinol.
